(19) Europäisches Patentamt
European Patent Office
Office européen des brevets

(11) EP 2 831 282 B1

(12) **EUROPEAN PATENT SPECIFICATION**

(45) Date of publication and mention of the grant of the patent:
**04.07.2018 Bulletin 2018/27**

(21) Application number: **13769291.9**

(22) Date of filing: **27.03.2013**

(51) Int Cl.:
***C12Q 1/68*** *(2018.01)*

(86) International application number:
**PCT/US2013/034013**

(87) International publication number:
**WO 2013/148775 (03.10.2013 Gazette 2013/40)**

(54) **PREDICTIVE BIOMARKER USEFUL FOR CANCER THERAPY MEDIATED BY A CDK INHIBITOR**

DURCH EINEN CDK-HEMMER VERMITTELTER PRÄDIKTIVER BIOMARKER FÜR KREBSTHERAPIE

BIOMARQUEUR PRÉDICTIF UTILE POUR THÉRAPIE ANTICANCÉREUSE MÉDIÉE PAR UN INHIBITEUR DE CDK

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2012 US 201261618087 P**

(43) Date of publication of application:
**04.02.2015 Bulletin 2015/06**

(73) Proprietor: **Merck Sharp & Dohme Corp.**
**Rahway, NJ 07065-0907 (US)**

(72) Inventors:
- **BOOHER, Robert Nolan**
  **Davis, CA 95616 (US)**
- **HIRSCH, Heather A.**
  **Boston, MA 02115-5727 (US)**
- **ZAWEL, Leigh Scott**
  **Boston, MA 02115-5727 (US)**
- **FAWELL, Stephen Eric**
  **Boston, MA 02115-5727 (US)**

(74) Representative: **Hussain, Deeba et al**
**Merck Sharp & Dohme Limited**
**Hertford Road**
**Hoddesdon**
**Hertfordshire EN11 9BU (GB)**

(56) References cited:
**WO-A1-2010/096627** **WO-A1-2011/063309**
**WO-A1-2012/131594** **WO-A2-2004/087954**
**US-A1- 2010 227 838**

- **GEORG FELDMANN ET AL: "Cyclin-dependent kinase inhibitor Dinaciclib (SCH727965) inhibits pancreatic cancer growth and progression in murine xenograft models", CANCER BIOLOGY & THERAPY, vol. 12, no. 7, 1 October 2011 (2011-10-01), pages 598-609, XP055222971, US ISSN: 1538-4047, DOI: 10.4161/cbt.12.7.16475**
- **G. E. KONECNY ET AL: "Expression of p16 and Retinoblastoma Determines Response to CDK4/6 Inhibition in Ovarian Cancer", CLINICAL CANCER RESEARCH, vol. 17, no. 6, 28 January 2011 (2011-01-28), pages 1591-1602, XP055223034, US ISSN: 1078-0432, DOI: 10.1158/1078-0432.CCR-10-2307**
- **ANURAG SAXENA ET AL: "Mcl-1 and Bcl-2/Bax ratio are associated with treatment response but not with Rai stage in B-cell chronic lymphocytic leukemia", AMERICAN JOURNAL OF HEMATOLOGY, vol. 75, no. 1, 1 January 2004 (2004-01-01), pages 22-33, XP055223048, US ISSN: 0361-8609, DOI: 10.1002/ajh.10453**
- **G. J. GORES ET AL: "Selectively targeting Mcl-1 for the treatment of acute myelogenous leukemia and solid tumors", GENES & DEVELOPMENT, vol. 26, no. 4, 15 February 2012 (2012-02-15), pages 305-311, XP055042047, ISSN: 0890-9369, DOI: 10.1101/gad.186189.111**
- **ZHANG ET AL.: 'Mcl-1 is critical for survival in a subgroup of non-small-cell lung cancer cell lines' ONCOGENE vol. 30, 06 December 2011, pages 1963 - 1968, XP055165259**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- BOOHER ET AL.: 'Abstract 3063: Mcl-1 dependency is a predictive biomarker for apoptotic induction by short-term dinaciclib (SCH 727965) treatment' POSTER PRESENTATIONS - CELL CYCLE CONTROL 2, PROCEEDINGS: AACR 103RD ANNUAL MEETING 2012, CANCER RESEARCH vol. 72, no. 8, 15 April 2012, pages 1 - 2, XP055165263

## Description

FIELD OF THE INVENTION

The present invention relates generally to the identification of biomarkers whose expression levels are useful for predicting a patient's response to treatment with an anti-proliferative agent, particularly one that is responsive to a cyclin-dependent kinase (CDK) inhibitor. The expression level of the identified biomarker can be used to predict a patient presenting with a cancerous condition that is mediated by inhibition of apoptosis and likely to respond to treatment with a CDK inhibitor prior to dosing with the CDK inhibitor.

BACKGROUND OF THE INVENTION

Protein kinase inhibitors include kinases such as, for example, the inhibitors of the cyclin-dependent kinases (CDKs), mitogen activated protein kinase (MAPK/ERK), glycogen synthase kinase 3 (GSK3beta), and the like. Protein kinase inhibitors are described, for example, by M. Hale, et al., in WO02/22610A1 and by Y. Mettey, et al., J. Med. Chem., 2003, 46:222-236. The cyclin-dependent kinases are serine/threonine protein kinases, which are the driving force behind the cell cycle and cell proliferation. Individual CDK's, such as, CDK1, CDK2, CDK3, CDK4, CDK5, CDK6 and CDK7, CDK8 and the like, perform distinct roles in cell cycle progression and can be classified as either G1, S, or G2M phase enzymes. Uncontrolled proliferation is a hallmark of cancer cells, and misregulation of CDK function occurs with high frequency in many important solid tumors. CDK2 and CDK4 are of particular interest because their activities are frequently misregulated in a wide variety of human cancers. CDK2 activity is required for progression through G1 to the S phase of the cell cycle and CDK2 is one of the key components of the G1 checkpoint. Checkpoints serve to maintain the proper sequence of cell cycle events and allow the cell to respond to insults or to proliferative signals, while the loss of proper checkpoint control in cancer cells contributes to tumorgenesis.

The CDK2 pathway influences tumorgenesis at the level of tumor suppressor function (e.g., p52, RB, and p27) and oncogene activation (cyclin E). Many reports have demonstrated that both the coactivator, cyclin E, and the inhibitor, p27, of CDK2 are either over or under expressed, respectively, in breast, colon, non-small cell lung, gastric, prostate, bladder, non-Hodgkin's lymphoma, ovarian, and other cancers. Their altered expression has been shown to correlate with increased CDK2 activity levels and poor overall survival. This observation makes CDK2 and its regulatory pathways compelling targets for the development as therapeutic agents for anti-proliferative disorders, such as cancer.

Adenosine 5'-triphosphate (ATP) competitive small organic molecules, as well as peptides have been reported in the literature as CDK inhibitors for the potential treatment of cancers. See, for example, US Patent 6,413,974, which describes various CDK inhibitors and their relationship to various types of cancer.

Other CDK inhibitors are known. For example, flavopiridol, whose structure is shown below, is a nonselective CDK inhibitor that is undergoing human clinical trials, A. M. Sanderowicz, et al., J. Clin. Oncol., 1998, 16:2986-2999.

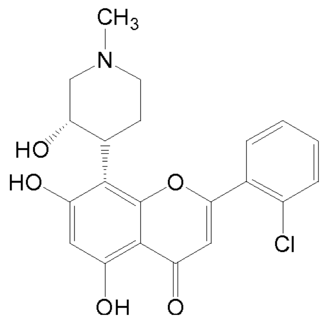

Other known CDK inhibitors include, for example, olomoucine (J. Vesely, et al., Eur. J. Biochem., 1994, 224:771-786) and roscovitine (L. Meijer, et al., Eur. J. Biochem., 1997, 243:527-536). U.S. Patent No. 6,107,305 describes certain pyrazolo[3,4-b] pyridine compounds as CDK inhibitors. K. S. Kim, et al., J. Med. Chem., 2002, 45:3905-3927 and WO 02/10162 disclose certain aminothiazole compounds as CDK inhibitors.

Myeloid cell leukemia (MCL)-1 is an anti-apopototic BCL-2 family member that possesses characteristics that make it potentially useful as a predictive biomarker for anti-proliferative therapeutic agents, such as CDK inhibitors, for use in treating proliferative disorders such as cancer (A. Mandelin and R. Pope, Expert Opin. Ther. Targets, 2007, 11(3):363-373). In particular, MCL-1 has been reported to be highly expressed in human leukemias and lymphomas, and the suppression of MCL-1 is believed to promote apoptosis (Mandelin and Pope, 2007, 367-368). It has also been reported that increased MCL-1 expression was associated with failure to achieve remission after treatment with fludarabine and chlorambucil in patients with chronic lymphocytic leukemia (CLL) (F.T. Awan, et al., Blood, 2009, 113(3):535-537). GEORG FELDMANN ET AL, 2011 ("Cyclin-dependent kinase inhibitor Dinaciclib (SCH727965) inhibits pancreatic cancer growth and progression in murine xenograft models", CANCER BIOLOGY & THERAPY, vol. 12, no. 7, 1 October 2011 (2011-10-01), pages 598-609) discloses that Ki67 immunostaining can be used as a marker of response to dinaciclib (see page 602, column 1).

There is a need for biomarkers that can be used to predict which patients are amenable to treatment with specific therapies, particularly for patients who are non-responsive or become refractive to first line therapies. It is, therefore, an object of this invention to provide a predictive biomarker to select patients likely to respond to treatment with a CDK inhibitor.

SUMMARY OF THE INVENTION

The instant invention relates generally to the identification of a predictive biomarker whose expression level is useful for evaluating and classifying patients for treatment with a CDK inhibitor. In the invention the predictive biomarker, the ratio of MCL-1 :BCL-xL, is used for predicting the response of a patient diagnosed with cancer to treatment with a CDK inhibitor, wherein the CDK inhibitor is SCH 727965 (Dinaciclib). In another embodiment, the invention is a method for identifying a patient diagnosed with cancer predicted to be responsive to treatment with a CDK inhibitor, wherein the CDK inhibitor is SCH 727965 (Dinaciclib). Also described is a method for treating a patient diagnosed with a CDK associated cancer by administering a CDK inhibitor, wherein the cancer cells of said patient are characterized by an MCL-1 :BCL-xL ratio gene expression level that is above a pre-determined reference value. In certain aspects of this disclosure the pre-determined reference value is obtained from cells of patients who have not been diagnosed with cancer. The MCL-1-:BCL-xL ratio gene expression level is the mRNA level.

BRIEF DESCRIPTION OF THE DRAWINGS

Figure 1 is a graphic illustration of the multiple mechanisms by which the CDK inhibitor, SCH 727965 (Dinaciclib), acts against CDK/cyclin kinase complexes.

Figure 2 is a graphic illustration of the inhibition of RNAPII CTD phosphorylation by CDK inhibitors, such as, flavopiridol, SNS-032, and SCH 727965 (Dinaciclib), which inhibits transcription and decreases expression of short-lived anti-apoptotic and pro-oncogenic proteins that lead to apoptosis.

Figure 3 is a graphic illustration of the anti-proliferative activity of the CDK inhibitor, SCH 727965 (Dinaciclib), in a wide range of tumor cell lines.

Figure 4 is a graphic illustration showing the differential sensitivity (mean viability) of two cell lines to the CDK inhibitor, SCH 727965 (Dinaciclib), at varying time points for a responder.

Figured 5A and 5B are graphic illustrations of the differential sensitivity to short term exposure of a CDK inhibitor, SCH 727965 (Dinaciclib), as correlated with the MCL-1:BCL-xL mRNA ratio in an 8 hour (Figure 5A) (for three cell lines) or an 18 hour (Figure 5B) (for 23 tumor cell lines) viability assay. The percent viability is inversely correlated to the MCL-1 :BCL-xL mRNA ratio, wherein a high ratio correlates with the induction of apoptosis (H23 in Figure 5A).

Figures 6A and 6B are graphic illustrations of the down regulation of expression for MCL-1 mRNA in ovarian cancer cells treated with 100 nM of the CDK inhibitor, SCH 727965 (Dinaciclib). Figure 6A shows the mRNA levels of five genes analyzed hourly for 5 hours, while Figure 6A is a re-scaled plot for four of the less abundant transcripts of Figure 6A. MCL-1 mRNA levels decreased dramatically within 3 hours of the addition of the CDK inhibitor.

Figures 7A and 7B are illustrations of the Western blot analysis of the time course during continuous treatment (Figure 7A) and post washout (Figure 7B) for the CDK inhibitor, SCH 727965 (Dinaciclib).

Figure 8 is an illustration of the Western blot analysis of the differential PARP cleavage response to short term treatment with the CDK inhibitor, SCH 727965 (Dinaciclib). The higher MCL-1:BCL-xL ratio-expressing cell lines (left side of figure) exhibited higher cleaved PARP levels, i.e., a measure of apoptosis, and greater sensitivity to the CDK inhibitor.

Figures 9A and 9B are graphical illustrations showing that the induction of apoptosis correlates with the MCL-1:BCL-xL ratio when treated with the CDK inhibitor, SCH 727965 (Dinaciclib), both in terms of PARP cleavage (Figure 9A) and caspase-3/7 activation (Figure 9B).

Figure 10 is a graphical illustration showing that high MCL-1:BCL-xL ratio mRNA expression levels correlates with the sensitivity to a CDK inhibitor in 387 heme (leukemia and lymphoma) and solid tumor malignancy cell lines following 24 hours of treatment with SCH 727965 (Dinaciclib). The correlation coefficient (r) as shown was -0.41 (p=5e-17).

Figure 11 is an illustration of the immunoblot analysis of the induction of apoptosis by a CDK inhibitor, SCH 727965 (Dinaciclib) in a solid tumor xenograft panel for tumor samples with a high MCL-1:BCL-xL ratio. Xenograft pharmacodynamic analysis was done 6 hours post-dose (40 mg/kg) of the CDK inhibitor. Immunoblot analytes of the tumor extracts (two representative samples) are shown on the left side and the treatments are shown on the right side of the blot. Cell lines: H23 (non-small cell lung cancer); A2780 (ovarian); Colo-320DM (colon); 22Rv1 (prostate); JIMT-1 (breast cancer); MDA-MB-231 (breast cancer); SW480 (colon); and PC-3 (prostate).

DETAILED DESCRIPTION OF THE INVENTION

As recognized by various cancer researchers, it is becoming important to identify potential responder biomarker(s) useful in predicting the therapeutic efficacy of an anti-cancer agent, e.g., CDK inhibitor, particularly for use in clinical trials and for the design of treatment regimes. Analysis of expression responder biomarker(s) are considered to be more feasible and less burdensome for patients, because the number of samples needed for the analysis are smaller as compared with conventional biomarker analysis, such as, the detection of protein phosphorylation with immunohistochemistry or DNA sequencing to detect a genetic alteration.

The present invention relaters to the discovery

of a responder biomarker for the selective CDK inhibitor, SCH 727965 (Dinaciclib), which has utility in predicting a patient's response to a treatment protocol comprising a CDK inhibitor. Applicants found that the level of gene expression of MCL-1 and BCL-xL (also known as BCL2L1), and specifically the level of expression for MCL-1 relative to BCL-xL, referred to herein as the MCL-1:BCL-xL ratio, was predictive of an apoptotic response to the selective CDK inhibitor, SCH 727965 (Dinaciclib). This correlation, i.e., SCH 727965 (Dinaciclib) sensitivity and high levels of expression of the MCL-1:BCL-xL ratio was observed in heme malignancy (leukemia and lymphoma) and solid tumor malignancy cell lines, such as, lung, breast, prostate, colorectal and ovarian tumor cell lines.

Definitions

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or."

"CDK inhibitor" means any compound or agent that inhibits the activity of one or more CDK proteins or CDK/cyclin kinase complexes. The compound or agent may inhibit CDK activity by direct or indirect interaction with a CDK protein or it may activity act to prevent expression of one or more CDK genes. Examples of small molecule CDK inhibitors are described above. In addition, the mechanism of the CDK inhibitors, flavopiridol and SNS-032, are described in R. Chen, et al., Blood, 2005, 106(7):2513-2519 and R. Chen, et al., Blood, 2009, 113 (19):4637-4645, respectively. The CDK Inhibitor, SCH 727965 (Dinaciclib), that is the subject of the studies herein, is described in D. Parry, et al., Mol. Cancer Ther., 2010 9(8):2344-235, W. Fu et al., Mol. Cancer Ther., 2011, 10(6):1018-1027, and U.S. Pat. 7,119,200, which are incorporated herein by reference as if set forth at length.

"Gene marker" or "marker" means an entire gene, or a portion thereof, such as an EST derived from that gene, the expression or level of which changes between certain conditions. Where the expression of the gene correlates with a certain condition, for example a drug treatment or a disease state, the gene is a marker for that condition.

"Predictive biomarker" means a gene marker whose expression is correlated with a response to a given therapeutic agent or class of therapeutic agents. As used herein, the term refers to myeloid cell leukemia (MCL)-1 and BCL-xL, whose expression ratio is correlated with the therapeutic effect of a CDK inhibitor. The CDK inhibitor is SCH 727965 (Dinaciclib).

"Marker-derived polynucleotides" means the RNA transcribed from a marker gene, any cDNA or cRNA produced therefrom, and any nucleic acid derived therefrom, such as synthetic nucleic acid having a sequence derived from the gene corresponding to the marker gene.

As used here, the terms "control," "control level," "reference level" or "pre-determined reference level" means a separate baseline level measured in a comparable control cell, which may or may not be disease free. It may be from the same individual or from another individual who is normal or does not present with the same disease from which the disease or test sample is obtained. Thus, "reference value" can be an absolute value, a range of values, an average value, a median value, a mean value, or a value as compared to a particular control or baseline value. A reference value can be based on an individual sample value, such as, a value obtained from a sample from an individual with a CDK mediated cancer, such as a solid tumor malignancy, but at an earlier point in time or prior to treatment, or a value obtained from a sample from a patient diagnosed with a CDK mediated cancer other than the individual being tested, or a "normal" individual, that is an individual not diagnosed with a CDK mediated cancer. The reference value can be based on a large number of samples, such as from patients diagnosed with a CDK mediated cancer, or normal individuals, or based on a pool of samples including or excluding the sample to be tested.

A "similarity value" is a number that represents the degree of similarity between two things being compared. For example, a similarity value may be a number that indicates the overall similarity between a patient's gene expression level using specific phenotype-related markers and a control specific to that phenotype (for instance, the similarity to a "good prognosis" reference level, where the phenotype is a good prognosis). The similarity value may be expressed as a similarity metric, such as a correlation coefficient, or may simply be expressed as the expression level difference, or the aggregate of the expression level differences, between a patient sample and a reference level.

As used herein, the terms "measuring expression levels," "measuring gene expression level," or "obtaining an expression level" and the like, includes methods that quantify target gene expression level exemplified by a transcript of a gene, including microRNA (miRNA) or a protein encoded by a gene, as well as methods that determine whether a gene of interest is expressed at all. Thus, an assay which provides a "yes" or "no" result without necessarily providing quantification of an amount of expression is an assay that "measures expression" as that term is used herein. Alternatively, the term may include quantifying expression level of the target gene expressed in a quantitative value, for example, a fold-change in expression, up or down, relative to a control gene or relative to the same gene in another sample, or a log ratio of expression, or any visual representation thereof, such as, for example, a "heat-map" where the

color intensity is representative of the amount of gene expression detected. Exemplary methods for detecting the level of expression of a gene include, but are not limited to, Northern blotting, dot or slot blots, reporter gene matrix (see, for example, US 5,569,588), nuclease protection, RT-PCR, microarray profiling, differential display, SAGE (Velculescu, et al., Science, 1995, 270:484-87), Digital Gene Expression System (see, WO2007076128; WO2007076129), multiplex mRNA assay (Tian, et al., Nucleic Acids Res., 2004, 32:e126), PMAGE (Kim, et al., Science, 2007, 316:1481-84), cDNA-mediated annealing, selection, extension and ligation assay (DASL, Bibikova, et al., AJP, 2004, 165:1799-807), multiplex branched DNA assay (Flagella, et al., Anal. Biochem., 2006, 352:50-60), 2D gel electrophoresis, SELDI-TOF, ICAT, enzyme assay, antibody assay, and the like.

As used herein, "subject" refers to an organism or to a cell sample, tissue sample or organ sample derived therefrom, including, for example, cultured cell lines, biopsy, blood sample or fluid sample containing a cell. In many instances, the subject or sample derived there from, comprises a plurality of cell types. In one embodiment, the sample includes, for example, a mixture of tumor cells and normal cells. In one embodiment, the sample comprises at least 10%, 15%, 20%, et seq., 90%, or 95% tumor cells. In one embodiment, the organism is a mammal, such as, a human, canine, murine, feline, bovine, ovine, swine, or caprine. In a particular embodiment, the organism is a human patient.

"Patient" as that term is used herein, refers to the recipient in need of medical intervention or treatment. Mammalian and non-mammalian patients are included. In one embodiment, the patient is a mammal, such as, a human, canine, murine, feline, bovine, ovine, swine, or caprine. In a particular embodiment, the patient is a human.

The term "treating" in its various grammatical forms in relation to the present invention refers to preventing (i.e. chemoprevention), curing, reversing, attenuating, alleviating, minimizing, suppressing or halting the deleterious effects of a disease state, disease progression, disease causative agent (e.g., bacteria or viruses) or other abnormal condition. For example, treatment may involve alleviating a symptom (i.e., not necessary all symptoms) of a disease or attenuating the progression of a disease.

"Treatment of cancer", as used herein, refers to partially or totally inhibiting, delaying or preventing the progression of cancer including cancer metastasis; inhibiting, delaying or preventing the recurrence of cancer including cancer metastasis; or preventing the onset or development of cancer (chemoprevention) in a mammal, for example a human. In addition, the methods of the present invention may be practiced for the treatment of chemoprevention of human patients with cancer. However, it is also likely that the methods would also be effective in the treatment of cancer in other mammals.

As used herein, the term "therapeutically effective amount" is intended to qualify the amount of the treatment in a therapeutic regimen necessary to treat cancer. This includes combination therapy involving the use of multiple therapeutic agents, such as a combined amount of a first and second treatment where the combined amount will achieve the desired biological response. The desired biological response is partial or total inhibition, delay or prevention of the progression of cancer including cancer metastasis; inhibition, delay or prevention of the recurrence of cancer including cancer metastasis; or the prevention of the onset or development of cancer (chemoprevention) in a mammal, for example a human.

As used herein, the terms "combination treatment", "combination therapy", "combined treatment" or "combinatorial treatment", used interchangeably, refer to a treatment of an individual with at least two different therapeutic agents. According to the invention, the individual is treated with a first therapeutic agent, preferably a DNA damaging agent and/or a CDK inhibitor as described herein. The second therapeutic agent may be another CDK inhibitor or may be any clinically established anti-cancer agent as defined herein. A combinatorial treatment may include a third or even further therapeutic agent.

"Status" means a state of gene expression of a set of genetic markers whose expression is strongly correlated with a particular phenotype. For example, "p53 status" means a state of gene expression of a set of genetic markers whose expression is strongly correlated with that of p53 gene, wherein the pattern of these genes' expression differs detectably between tumors expressing the protein and tumors not expressing the protein.

"Good prognosis" means that a patient is expected to have no distant metastases of a tumor within five years of initial diagnosis of cancer.

"Poor prognosis" means that a patient is expected to have distant metastases of a tumor within five years of initial diagnosis of cancer.

Embodiment(s) of the Invention

A broad aspect of the disclosure concerns the identification of a predictive biomarker, the MCL-1 :BCL-xL ratio, whose level is correlated with a response to a CDK inhibitor, that can be used to identify patients likely to respond to treatment with a CDK inhibitor. The CDK inhibitor is SCH 727965 (Dinaciclib). In another aspect the disclosure is a method to treat patients diagnosed with cancer, in particular a CDK mediated cancer, with a CDK inhibitor, comprising administering to the cancer patient a compound which is a CDK inhibitor, wherein the cancer cells of said patient are characterized by a MCL-1 :BCL-xL ratio gene expression level that is above a predetermined reference value. The MCL-1 :BCL-xL ratio gene expression level is the mRNA level.

MCL-1:BCL-xL ratio as a predictive biomarker for a CDK inhibitor

SCH 727965 (Dinaciclib) is a potent and selective inhibitor of the cyclin-dependent kinases (CDKs) 1, 2, 5 and 9 undergoing clinical testing against a range of solid and hematologic malignancies. During preclinical studies more than 140 cell lines have been profiled for the response of SCH 727965 (Dinaciclib) in long-term (≥ 72 hours) viability or clonogenicity assays, wherein greater than 97% of the lines exhibited an IC50 ≤ 25 nM. Without wishing to be bound to any theory, Applicants believe that this uniformly low nM potency was likely due to the repression of both cell cycle progression and transcription, through the inhibition of CDK1/2 and CDK9, respectively. CDK9 phosphorylation of the RNA pol II (RNAPII) at Ser2 and Ser5 is required for transcriptional initiation and elongation. Applicants and others have observed rapid CDK9-dependent effects on cells after short-term exposure to CDK inhibitors, such as, SCH 727965 (Dinaciclib), including the loss of RNAPII Ser2 phosphorylation, followed by the rapid elimination of the short half-life, pro-survival protein MCL-1 (Chen, et al., Blood, 2005, 106:2513-2519, Chen, et al., Blood, 2009, 113:4637-4645).

The ability of a cancer cell to avoid apoptosis is believed to be dependent on the balance of several Bcl-2 anti-apoptotic family members, which include BCL-2, BCL-xL, BCL-w and MCL-1. As such, Applicants hypothesized that MCL-1 dependent cell lines would be more sensitive to treatment with a selective CDK inhibitor, such as, SCH 727965 (Dinaciclib). Applicants further hypothesized that the differential sensitivity of SCH 727965 (Dinaciclib) could be discriminated from the longer-term inhibitory cell cycle effects by conducting short term SCH 727965 (Dinaciclib) exposure assays.

Applicants have now found that the activity of a selective CDK inhibitor, such as, SCH 727965 (Dinaciclib), induced apoptosis in a panel of 25 human solid tumor cell lines with varying levels of MCL-1 dependency. MCL-1 dependency in solid tumor cell lines has been reported to correlate with the MCL-1:BCL-xL mRNA ratio or the level of MCL-1 gene amplification (Beroukhim, et al., Nature, 2010, 463:899-905; Zhang, et al., Oncogene, 2011, 30:1963-1968). Applicants assessed cell viability after 18 hours of treatment with 100 nM of SCH 727965 (Dinaciclib) (Figure 5B), while target engagement and induction of apoptosis was determined after 8 hours (Figure 5A). With one exception, all cell lines showed potent CDK9 target engagement, as determined by the loss of RNAPII Ser2 phosphorylation and the corresponding reduced MCL-1 protein levels (Figure 8). Applicants observed that the loss of cell viability, measured by ATP content, directly correlated with the MCL-1:BCL-xL mRNA ratio (Figures 5A, 10, and 11). A dramatic increase in PARP cleavage was also observed in cell lines with the highest MCL-1:BCL-xL mRNA ratio (Figures 8 and 9A). Further, the extent of PARP cleavage correlated with the level of caspase-3 or caspase-7 activity (Figure 9B), while the level of BCL-2 (Figure 8) did not significantly impact the response of SCH 727965 (Dinaciclib). Thus, either at the mRNA level (Figures 5A and 5B) or at the protein level (Figure 8), high ratio of MCL-1:BCL-xL gene expression, equivalent to high levels of apoptosis, correlates with sensitivity to SCH 727965 (Dinaciclib). This relationship was confirmed in a xenograph pharmacodynamics analysis where the CDK inhibitor, SCH 727965 (Dinaciclib) induced apoptosis in lung, ovarian, colon, and prostate tumor cell lines having high expression of the MCL-1:BCL-xL ratio (Figure 11).

Taken together, these data demonstrate that the MCL-1:BCL-xL ratio can be used as a predictive biomarker to identify patients, diagnosed with a solid tumor or hematological malignancy, such as chronic lymphocytic leukemia (CLL), that are likely to respond to treatment with a selective CDK inhibitor, such as, SCH 727965 (Dinaciclib).

CDK Inhibitors

Cyclin-dependent kinases (CDK) are key positive regulators of cell cycle progression and are attractive targets in oncology. However, due to the high degree of structural homology within the CDK protein family, putative small molecule CDK inhibitors may exert their effects through combinatorial inhibition of multiple CDKs and other closely related serine/threonine kinases, resulting in adverse effects attributable to non-specific inhibition (D. Parry, et al., Mol. Cancer Ther., 2010, 9(8):2344-2353).

SCH 727965 (Dinaciclib), the structure of which is shown below, has previously been shown to inhibit CDK2, CDK5, CDK1, and CDK9 with $IC_{50}$ values of 1, 1, 3, and 4 nM, respectively (Parry, *et al.,* 2010, 2347).

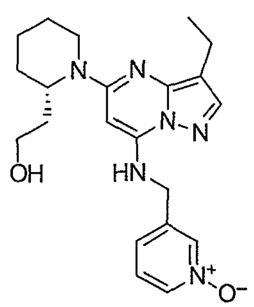

As compared to flavopiridol, SCH 727965 (Dinaciclib) is an equally potent inhibitor of CDK1 and CDK9, but is a 12-fold and 14-fold stronger inhibitor of CDK2 and CDK5, respectively. The compound was also found to be a potent DNA replicator inhibitor the blocked thymidine DNA incorporation in A2780 cells with an $IC_{50}$ of 4 nM (Id.). Taken together, SCH 727965 (Dinaciclib) is a stronger and more selective inhibitor of CDKs, that translates into its more potent inhibition of DNA synthesis as compared to flavopiridol (data not shown) (Id.).

As shown in Table 1 below, D. Parry, et al., Mol. Cancer Ther., 2010, 9(8):2344-2353, found that the $IC_{50}$ values (nM) for SCH 727965 (Dinaciclib) demonstrated that this CDK inhibitor was most active against CDKs 1, 2, 5, and 9, while it was at least 10 to 100 fold less active against CDKs 4, 6, and 7. SCH 727965 (Dinaciclib) had greater than 10 $\mu$M $IC_{50}$ against a Millipore panel of 50 diverse kinases. Thus, it is evident that SCH 727965 (Dinaciclib), acts through multiple mechanisms (Figure 1).

Table 1

| CDK/Cyclin Kinase Complex | $IC_{50}$ Value (nM) |
|---|---|
| Cdk2/E | 1 |
| Cdk2/A | 1 |
| Cdk1/B1 | 3 |
| Cdk4/D1 | 100 |
| Cdk5/p25 | 1 |
| Cdk6/D3 | 60 |
| Cdk7/H | 70 |
| Cdk9/T | 4 |

CDK inhibitors, such as, flavopiridol, SNS-032, and SCH 727965 (Dinaciclib), are known to act through CDK 7 and 9, which leads to inhibition of RNAP II CTD phosphorylation (R. Chen, et al., Blood, 2005, 106 (7):2513-2516; R. Chen, et al., Blood, 2009, 113(19):4637-4645; W. Fu, et al., Mol. Cancer Ther., 2011, 10(6): 1018-1027). Inhibition of RNAP II CTD phosphorylation in turn has been shown to inhibit transcription and decrease expression of short lived anti-apototic and pro-oncogenic proteins, such as MCL-1 and C-Myc and Cyclin D, respectively (Figure 2).

More specifically, treatment with the CDK inhibitor, SCH 727965 (Dinaciclib), exhibited potent anti-proliferative activity against a wide range of cell lines (Figure 3). The cellular potency of SCH 727965 (Dinaciclib) resulted in 50% growth inhibition ($EC_{50}$) in a 96 hour viability assay conducted over 511 tumor cell lines. SCH 727965 (Dinaciclib) exhibited $EC_{50}$s in the 5-45 nM range for all cell lines and an $EC_{50}$< 20 nM in more than 80% of the cell lines evaluated.

The CDK inhibitor, SCH 727965 (Dinaciclib), also demonstrated differential sensitivity at 24 hours as compared to 96 hours (Figure 4). In Figure 4, Cell Line A would be representative of a responder at time points greater than 4 hours at the concentration of interest, while Cell Line B would be representative of a responder at 72 hours and 96 hours, but not at 24 hours. Without wishing to be bound by any theory, the differential response to SCH 727965 (Dinaciclib) at an early time point suggests that a non-cell cycle mechanism influences sensitivity during short exposures to the therapeutic agent. This indicates that even short-term exposure with a CDK inhibitor, such as SCH 727965 (Dinaciclib), may have a differential effect on cancer cells depending on the expression of a specific biomarker.

Moreover, the differential cell line sensitivity observed for short term exposure of SCH 727965 (Dinaciclib) (Figure 4) correlates with the MCL-1:BCL-xL mRNA ratio (Figures 5A and 5B). In Figure 5B, lung, breast, prostate, colorectal and ovarian cell lines were treated with 100 nM of the CDK inhibitor, SCH 727965 (Dinaciclib), for 18 hours and assayed for viability by ATP quantitation.

Having observed that the CDK inhibitor, SCH 727965 (Dinaciclib), exhibited anti-proliferative activity in a wide range of tumor cell lines and that the differential short term exposure sensitivity correlated the MCL-1:BCL-xL ratio, Applicants sought to determine what effect SCH 727965 (Dinaciclib) would have on the specific expression of various genes known to be associated with apoptosis, including MCL-1, Cyclin E1, BCL2L2, BCL-xL, and BCL-2. Ovarian cancer cells (A2780) were treated with 100 nM of SCH 727965 (Dinaciclib) and the mRNA expression levels of the five genes were analyzed hourly for 5 hours. It was found that SCH 727965 (Dinaciclib) specifically down-regulated MCL-1 (Figure 6A).

Similarly, a Western blot analysis was carried out for various markers associated with apoptosis in ovarian cancer cells (A2780) treated continuously over a 5 hour time course with 100 nM of SCH 727965 (Dinaciclib) (Figure 7A). Applicants observed the rapid reduction in expression of the CDK9 phosphorylation site in RNAP II CTD, the decrease in expression of the short-lived MCL-1 and c-MYC proteins, and the induction of apoptosis as measured by cleaved PARP. Conversely, in a wash-out analysis, treatment with the CDK inhibitor, SCH 727965 (Dinaciclib), resulted in the rapid reversal of CDK9 inhibition and MCL-1 levels and induced some apoptosis as measured by cleaved PARP (Figure 7B). In the wash-out assay, 100 nM of SCH 727965 (Dinaciclib) was added to A2780 cells at inception (t=0), washed-out from three samples after a 2 hour exposure, and then analyzed at 4, 6, and 8 hours after t=0.

Based on this short term differential response of the CDK inhibitor, SCH 727965 (Dinaciclib), Applicants then evaluated apoptotic induction as measured by PARP cleavage. Lung, breast, prostate, colorectal and ovarian cell lines were treated with 100 nM of SCH 727965 (Dinaciclib) or DMSO for 8 hours. In a Western blot analysis Applicants observed that apoptotic induction, as measured by PARP cleavage (Figure 8), was analogous to the differential sensitivity observed in the 18 hour viability assay (Figure 5B), which correlated with the MCL-1:BCL-xL ratio. MCL-1 mRNA expression rapidly declined, while the level of BCL-2 expression was relatively unchanged, suggesting that it was MCL-1 and not BCL-2 that affected the response observed, i.e. induction of apoptosis, for the CDK inhibitor, SCH 727965 (Dinaciclib).

Similarly, the induction of apoptosis observed

for SCH 727965 (Dinaciclib) correlated with the MCL-1:BCL-xL ratio (Figures 9A and 9B). In Figure 9A, PARP cleavage versus the MCL-1:Bcl-xL ratio was quantified from a Western blot analysis of 27 lung, breast, prostate, colorectal and ovarian cell lines treated with 100 nM of SCH 727965 (Dinaciclib) for 18 hours. In Figure 9B, the time course of caspase-3/7 activation during a 24 hour period was measured after treatment of lung, breast, prostate, colorectal and ovarian cell lines with SCH 727965 (Dinaciclib). Consistent with PARP cleavage, the activation of caspase-3/7 activation correlated with the MCL-1:BCL-xL ratio.

That high MCL1-BCL-xL mRNA expression ratio correlates with sensitivity to a CDK inhibitor was also evident in Figure 10, where the percentage of cell viability remaining after 24 hours of treatment is shown for 387 heme (leukemia and lymphoma) and solid malignancy cell lines. This relationship observed *in vitro* was retained *in vivo* in a solid tumor xenograft panel, where the CDK inhibitor, SCH 727965 (Dinaciclib), induced apoptosis in high MCL-1:BCL-xL ratio lung, ovarian, colon, and prostate tumors (Figure 11). Xenograft pharmacodynamic analysis 6 hours post-dosing (40 mg/kg) with the CDK inhibitor, SCH 727965 (Dinaciclib), showed that the CDK inhibitor induced apoptosis (cleaved PARP) in high MCL-1:BCL-xL ratio tumors.

Thus, Applicants have shown that SCH 727965 (Dinaciclib) is a selective inhibitor of CDKs 1, 2, 5, and 9, known to be associated with apoptosis. In long term (> 72 hours) treatment protocols, SCH 727965 (Dinaciclib) exhibits high potency (EC50s of 5-45 nM) in a panel of greater than 500 cell lines across a wide range of tumor types. Short-term (8-24 hours) treatment with SCH 727965 (Dinaciclib) shows differential viability and apoptotic responses that correlate with the MCL-1:BCL-xL ratio. SCH 727965 (Dinaciclib) inhibits CDK9, which in turn reduces CTD Ser2 phosphorylation of RNA Pol II and decreases the expression of MCL-1 and c-MYC. Taken together, one of ordinary skill in the art would readily acknowledge and appreciate that the MCL-1:BCL-xL ratio can be used as a predictive biomarker for an anti-cancer response in solid tumor malignancies.

Classification of a cell sample having sensitivity to a CDK inhibitor

Identification of a predictive biomarker

The present invention provides a biomarker whose level co-relates with a response to a CDK inhibitor. Generally, the biomarker was identified as detailed in the Examples set forth below by determining which of the apoptotic genes had expression patterns that correlated with the treatment response.

Sample Collection

In the present invention, target polynucleotide molecules are extracted from a sample taken from an individual afflicted with a cancer. The sample may be collected in any clinically acceptable manner, but must be collected such that marker-derived polynucleotides (i.e., RNA) are preserved. mRNA or nucleic acids derived therefrom (i.e., cDNA or amplified DNA) are preferably labeled distinguishably from standard or control polynucleotide molecules, and both are simultaneously or independently hybridized to a microarray comprising some or all of the markers or marker sets or subsets described above. Alternatively, mRNA or nucleic acids derived therefrom may be labeled with the same label as the standard or control polynucleotide molecules, wherein the intensity of hybridization of each at a particular probe is compared. A sample may comprise any clinically relevant tissue sample, such as a tumor biopsy or fine needle aspirate, or a sample of bodily fluid, such as blood, plasma, serum, lymph, ascitic fluid, cystic fluid, urine or nipple exudate. The sample may be taken from a human, or, in a veterinary context, from non-human animals such as ruminants, horses, swine or sheep, or from domestic companion animals such as felines and canines.

Methods for preparing total and poly(A)+RNA are well known and are described generally in Sambrook et al., MOLECULAR CLONING--A LABORATORY MANUAL (2ND ED.), Vols. 1 3, Cold Spring Harbor Laboratory, Cold Spring Harbor, NY, 1989) and Ausubel, et al., CURRENT PROTOCOLS IN MOLECULAR BIOLOGY, vol. 2, Current Protocols Publishing, New York, 1994).

RNA may be isolated from eukaryotic cells by procedures that involve lysis of the cells and denaturation of the proteins contained therein. Cells of interest include wild-type cells (i.e., non-cancerous), drug-exposed wild-type cells, tumor- or tumor-derived cells, modified cells, normal or tumor cell line cells, and drug-exposed modified cells.

Additional steps may be employed to remove DNA. Cell lysis may be accomplished with a nonionic detergent, followed by microcentrifugation to remove the nuclei and hence the bulk of the cellular DNA. In one embodiment, RNA is extracted from cells of the various types of interest using guanidinium thiocyanate lysis followed by CsCl centrifugation to separate the RNA from DNA (Chirgwin, et al., Biochemistry, 1979, 18:5294-5299). Poly(A)+RNA is selected by selection with oligo-dT cellulose (see, Sambrook, et al., MOLECULAR CLONING--A LABORATORY MANUAL (2ND ED.), Vols. 1 3, Cold Spring Harbor Laboratory, Cold Spring Harbor, NY, 1989). Alternatively, separation of RNA from DNA can be accomplished by organic extraction, for example, with hot phenol or phenol/chloroform /isoamyl alcohol. If desired, RNAse inhibitors may be added to the lysis buffer. Likewise, for certain cell types, it may be desirable to add a protein denaturation/digestion step to the protocol.

For many applications, it is desirable to preferentially enrich mRNA with respect to other cellular RNAs, such as transfer RNA (tRNA) and ribosomal RNA (rRNA).

Most mRNAs contain a poly(A) tail at their 3' end. This allows them to be enriched by affinity chromatography, for example, using oligo(dT) or poly(U) coupled to a solid support, such as cellulose or SEPHADEX.RTM. medium (see Ausubel et al., CURRENT PROTOCOLS IN MOLECULAR BIOLOGY, vol. 2, Current Protocols Publishing, New York, 1994). Once bound, poly(A)+mRNA is eluted from the affinity column using 2 mM EDTA/0.1% SDS.

The sample of RNA can comprise a plurality of different mRNA molecules, each different mRNA molecule having a different nucleotide sequence. In a specific embodiment, the mRNA molecules in the RNA sample comprise at least 100 different nucleotide sequences. More preferably, the mRNA molecules of the RNA sample comprise mRNA molecules corresponding to each of the marker genes. In another specific embodiment, the RNA sample is a mammalian RNA sample.

In a specific embodiment, total RNA or mRNA from cells are used in the methods of the invention. The source of the RNA can be cells of a plant or animal, human, mammal, primate, non-human animal, dog, cat, mouse, rat, bird, yeast, eukaryote, prokaryote, etc. In specific embodiments, the method of the invention is used with a sample containing total mRNA or total RNA from 1.times.10.sup.6 cells or less. In another embodiment, proteins can be isolated from the foregoing sources, by methods known in the art, for use in expression analysis at the protein level.

Prediction of sensitivity/resistance of a cell sample to CDK inhibitor treatment

The invention provides a gene marker, the MCL-1:BCL-xL ratio, whose expression is correlated with a subject's response to a treatment with a CDK inhibitor. The CDK inhibitor is dinaciclib, and the ratio is of the mRNA level. The invention also provides a method of using this predictive biomarker to select a patient for treatment with the CDK inhibitor in diagnosis or pre-dose prediction.

The predictive biomarker, the MCL-1:BCL-xL ratio, provided herein may also be used in combination with other markers for CDK mediated disorders such as cancer, such as solid tumor malignancies, or for any other clinical or physiological condition for which CDK is associated. The present invention provides a marker, the MCL-1:BCL-xL ratio, which can be used to predict a cell sample as having sensitivity to a biologically active dose of a CDK inhibitor, in particular to the CDK inhibitor, SCH 727965 (Dinaciclib). In some instances it is of value to determine if a particular cell population is sensitive or resistant to a therapeutic dose of a CDK inhibitor.

In some embodiments, the predictive biomarker is evaluated relative to a pre-determined reference value, wherein the pre-determined reference value is based upon the biomarker gene expression measurements taken in control samples exposed to a CDK inhibitor. The pre-determined reference value may be expressed in several way, including, but not limited to, a fold change, up or down, of 1.2-fold change or greater, 1.3-fold or greater or 1.4-fold or greater, or 1.5-fold or greater, 1.6-fold or greater, 1.7-fold or greater, 1.8-fold or greater, 1.9-fold or greater, 2.0-fold or greater, or 3.0-fold or greater. The 2-fold means 2-fold up-regulated or 1/2-fold down-regulated of the markers in CDK inhibitor treated samples compared with non-treated control samples. In one embodiment the control sample is from a non-cancerous patient or individual and the pre-determined reference value is at least 1 to 2 fold up-regulated, or any fold level in between, in the cancer patient or individual sample, i.e. a non-control sample, as compared to that from a non-cancerous patient or individual sample. In one embodiment the control sample is from a non-cancerous patient and the pre-determined reference value is a least 1 to 2 fold up-regulated in the cancer patient sample as compared to that from a non-cancerous patient sample. In still another embodiment the control sample is from a sample from a patient or individual not diagnosed with a CDK mediated cancer and the pre-determined reference value is at least 1 to 2 fold up-regulated, or any fold level in between, as compared to that from a patient or individual diagnosed with a CDK mediated cancer.

In another aspect of the disclosure a predictive biomarker and methods are provided that are useful in predicting sensitivity and/or resistance of a subject to treatment with a CDK inhibitor. The predictive biomarker is used to make a drug response prediction based upon gene expression levels measured in a cell sample comprising tumor cells before CDK inhibitor treatment. The expression level of the prediction biomarker, the MCL-1:BCL-xL ratio, is correlated with sensitivity of cells to CDK inhibitor treatment. Biological samples in which the MCL-1:BCL-xL ratio level is increased relative to a control prior to treatment with a CDK inhibitor are those expected to be sensitive to treatment with a CDK inhibitor.

CDK inhibitor sensitivity or resistance is predicted in a subject using the predictive biomarker, the MCL-1:BCL-xL ratio. One aspect of the present invention provides a method of using this CDK inhibitor predictive biomarker to predict whether a subject with cancer will respond to treatment with a CDK inhibitor. In another aspect, the invention provides a method of using the CDK inhibitor predictive biomarker to predict whether a subject with cancer will respond to treatment with SCH727965 (Dinaciclib).

In certain embodiments, the invention comprises using data obtained from the predictive biomarker as a means of determining whether a patient should continue treatment with a CDK inhibitor or to be treated with a CDK inhibitor in the first place. Thus, biological samples from patients exhibiting a favorable data set, e.g., those that are sensitive, may continue treatment with the CDK inhibitor or start treatment with a CDK inhibitor. The methods of the invention may also be used to stratify a patient population into a treatment group, e.g., those that can be

treated with a CDK inhibitor and thus may be enrolled into a therapeutic regiment employing a CDK inhibitor or a non-treatment group, e.g., those that are not amenable to treatment with a CDK inhibitor. Towards this end, the methods of the invention may also be used to identify patients who may need to be pulled out of a therapeutic protocol comprising a CDK inhibitor where the biomarker data is not positive, e.g., those that are resistant or non-sensitive. Also disclosed is a method which comprises:

(a) calculating a measure of similarity between a first gene expression level from a cell sample and a CDK inhibitor sensitive (responder) reference level, or calculating the measure of similarity between said first gene expression level and said CDK inhibitor sensitive (responder) reference level and a second measure of similarity between said first gene expression level and a CDK inhibitor resistant (non-responder) reference level, said first gene expression level comprising a measured expression level of the predictive biomarker in a cell sample obtained from a subject, wherein said cell sample comprises cancer cells and is obtained from said subject prior to treatment of said subject with a CDK inhibitor, said CDK inhibitor sensitive (responder) reference level comprising a measured expression level of said predictive biomarker that is the average expression level of the biomarker in a first plurality of control cell samples that are sensitive to treatment with said CDK inhibitor, and said CDK inhibitor resistance (non-responder) reference level comprising a measured expression level of said predictive biomarker that is the average expression level of the biomarker in a second plurality of control cell samples that are resistant to treatment with said CDK inhibitor;

(b) predicting that said subject will:

(i) be sensitive to CDK inhibitor treatment if said first gene expression level has high similarity to said CDK inhibitor sensitive (responder) reference level or has higher similarity to said CDK inhibitor sensitive (responder) reference level than to said CDK inhibitor resistant (non-responder) reference level, or

(ii) be resistant to CDK inhibitor treatment if said first gene expression level has low similarity to said CDK inhibitor sensitive (responder) reference level or has higher similarity to said CDK inhibitor resistant (non-responder) reference level than to said CDK inhibitor sensitive (responder) reference level;

wherein said first gene expression level has a high similarity to said CDK inhibitor sensitive (responder) reference level if the similarity to said CDK inhibitor sensitive (responder) reference level is above a pre-determined threshold or reference value, or has a low similarity to said CDK inhibitor sensitive (responder) reference level if the similarity to said CDK inhibitor sensitive (responder) reference level is below said pre-determined threshold or reference value. The method further proposes treating the patient with a CDK inhibitor based upon the prediction, e.g., treating patients demonstrating a sensitive profile and pulling out patients from a treatment protocol comprising a CDK inhibitor if their expression level is that of a non-responder, e.g., non-responsive to a CDK inhibitor.

Similarity between a gene expression level and a sensitive/resistant reference value

The degree of similarity between a gene expression level obtained from a cell sample and a reference level can be determined using any method known in the art. For example, Dai *et al.,* describe a number of different ways of calculating gene expression levels and corresponding gene biomarkers useful in classifying breast cancer patients (U.S. Pat. 7,171,311; WO2002103320; WO200508689l; WO20060l5312; WO2006084272). Similarly, Linsley, et al., US 2003/0104426, and Radish, et al., US 20070154931, disclose gene biomarkers and methods of calculating gene expression levels useful in classifying chronic myelogenous leukemia patients.

In one embodiment, the reference or control comprises target polynucleotide molecules derived from a sample from a cell sample not exposed to the CDK inhibitor. In another embodiment, the reference or control is a pool of target polynucleotide molecules. The pool may be derived from collected samples from a number of cancer individuals. In certain embodiments, the pool comprises samples taken from a number of individuals having cancers responsive to a CDK inhibitor. In another embodiment, the pool comprises an artificially-generated population of nucleic acids designed to approximate the level of nucleic acid derived for the biomarker in a pool of biomarker-derived nucleic acids derived from tumor samples. In yet another embodiment, the pool is derived from cancer cell lines or cell line samples.

The comparison may be accomplished by any means known in the art. For example, expression levels of various markers may be assessed by separation of target polynucleotide molecules (e.g., RNA or cDNA) derived from the markers in agarose or polyacrylamide gels, followed by hybridization with marker-specific oligonucleotide probes. Alternatively, the comparison may be accomplished by the labeling of target polynucleotide molecules followed by separation on a sequencing gel. Polynucleotide samples are placed on the gel such that patient and control or standard polynucleotides are in adjacent lanes. Comparison of expression levels is accomplished visually or by means of densitometer. In one embodiment, the expression of the biomarker is assessed by hybridization to a microarray. In each approach, the biomarker is evaluated relative to that in a sample or control identified as associated with a cancer

responsive to a CDK inhibitor.

The expression of the identified CDK predictive biomarker may also be used to identify markers that can differentiate tumors into clinical types. In certain embodiments, beginning with a number of tumor samples, one may identify tumor specific markers by calculating the correlation coefficients between the clinical category or clinical parameter(s) and the linear, logarithmic or any transform of the expression ratio across all samples for each individual gene. Specifically, the correlation coefficient is calculated as:

$$\rho=(\vec{c}\cdot\vec{r})/(\|\vec{c}\|\cdot\|\vec{r}\|)$$

where $\vec{c}$ represents the clinical parameters or categories and $\vec{r}$ represents the linear, logarithmic or any transform of the ratio of expression between sample and control. Markers for which the coefficient of correlation exceeds a cutoff are identified as breast cancer-related markers specific for a particular clinical type. Such a cutoff or threshold corresponds to a certain significance of discriminating genes obtained by Monte Carlo simulations. The threshold depends upon the number of samples used; the threshold can be calculated as $3\times1/\sqrt{n-3}$, where $1/\sqrt{n-3}$ is the distribution width and n=the number of samples. In a specific embodiment, markers are chosen if the correlation coefficient is greater than about 0.3 or less than about -0.3.

Next, the significance of the correlation is calculated. This significance may be calculated by any statistical means by which such significance is calculated. In one example, a set of correlation data is generated using a Monte-Carlo technique to randomize the association between the expression difference of a particular marker and the clinical category. The frequency distribution of markers satisfying the criteria through calculation of correlation coefficients is compared to the number of markers satisfying the criteria in the data generated through the Monte-Carlo technique. The frequency distribution of markers satisfying the criteria in the Monte-Carlo runs is used to determine whether the number of markers selected by correlation with clinical data is significant.

Once a marker set is identified, the markers may be rank-ordered in order of significance of discrimination. One means of rank ordering is by the amplitude of correlation between the change in gene expression of the marker and the specific condition being discriminated. Another, preferred, means is to use a statistical metric. In one embodiment, the metric is a Fisher-like statistic:

$$t=(\langle x_1\rangle-\langle x_2\rangle)/\sqrt{[\sigma_1^2(n_1-1)+\sigma_2^2(n_2-1)]/(n_1+n_2-1)/(1/n_1+1/n_2)}$$

In this equation, $\langle x_1\rangle$ is the error-weighted average of the log ratio of transcript expression measurements within a first diagnostic group (e.g., ER(-), $\langle x_2\rangle$ is the error-weighted average of log ratio within a second, related diagnostic group (e.g., ER(+)), $\sigma_1$ is the variance of the log ratio within the ER(-) group and $n_1$ is the number of samples for which valid measurements of log ratios are available. $\sigma_2$ is the variance of log ratio within the second diagnostic group (e.g., ER(+)), and $n_2$ is the number of samples for which valid measurements of log ratios are available. The t-value represents the variance-compensated difference between two means.

The rank-ordered marker set may be used to optimize the number of markers in the set used for discrimination. This is accomplished generally in a "leave one out" method as follows. In a first run, a subset, for example 5, of the markers from the top of the ranked list is used to generate a template, where out of X samples, X-1 are used to generate the template, and the status of the remaining sample is predicted. This process is repeated for every sample until every one of the X samples is predicted once. In a second run, additional markers, for example 5, are added, so that a template is now generated from 10 markers, and the outcome of the remaining sample is predicted. This process is repeated until the entire set of markers is used to generate the template. For each of the runs, type 1 error (false negative) and type 2 errors (false positive) are counted; the optimal number of markers is that number where the type 1 error rate, or type 2 error rate, or preferably the total of type 1 and type 2 error rate is lowest.

For prognostic markers, validation of the marker set may be accomplished by an additional statistic, a survival model. This statistic generates the probability of cancer as measured by, for example, tumor burden as a function of time since initial diagnosis. A number of models may be used, including Weibull, normal, log-normal, log logistic, log-exponential, or log-Rayleigh (Chapter 12 "Life Testing", S-PLUS 2000 GUIDE TO STATISTICS, Vol. 2, p. 368 (2000)). For the "normal" model, the probability of distant metastases P at time t is calculated as

$$P=\alpha\times\exp(-t^2/\tau^2)$$

where $\alpha$ is fixed and equal to 1, and $\tau$ is a parameter to be fitted and measures the "expected lifetime".

See US Patent 7,171,311 for each of the above referenced equations. The entire content of the above patent is incorporated by reference herein.

It will be apparent to those skilled in the art that the above methods, in particular the statistical methods, described above, are not limited to the identification of markers associated with a CDK inhibitor or CDK mediated cancer, but may be used to identify set of marker genes associated with any phenotype. The phenotype can be the presence or absence of a disease such as cancer, or the presence or absence of any identifying clinical condition associated with that cancer. In the disease context, the phenotype may be a prognosis such as a survival time, probability of distant metastases of a disease condition, or likelihood of a particular response to a therapeutic or prophylactic regimen. The phenotype need not be cancer, or a disease; the phenotype may be a nominal characteristic associated with a healthy individual.

In one embodiment, the similarity is represented by a correlation coefficient between the sample profile and the template. In another embodiment, a correlation coefficient above a correlation threshold indicates high similarity, whereas a correlation coefficient below the threshold indicates low similarity. In some embodiments, the correlation threshold is set as 0.3, 0.4, 0.5 or 0.6. In another embodiment, similarity between a sample profile and a template is represented by a distance between the sample profile and the template. In one embodiment, a distance below a given value indicates high similarity, whereas a distance equal to or greater than the given value indicates low similarity.

Determination of marker gene expression levels

Methods

The expression levels of the marker genes in a sample may be determined by any means known in the art. The expression level may be determined by isolating and determining the level (i.e., amount) of nucleic acid transcribed from each marker gene. Alternatively, or additionally, the level of specific proteins encoded by a marker gene may be determined.

The level of expression of specific marker genes can be accomplished by determining the amount of mRNA, or polynucleotides derived therefrom, present in a sample. Any method for determining RNA levels can be used. For example, RNA is isolated from a sample and separated on an agarose gel. The separated RNA is then transferred to a solid support, such as a filter. Nucleic acid probes representing one or more markers are then hybridized to the filter by northern hybridization, and the amount of marker-derived RNA is determined. Such determination can be visual, or machine-aided, for example, by use of a densitometer. Another method of determining RNA levels is by use of a dot-blot or a slot-blot. In this method, RNA, or nucleic acid derived therefrom, from a sample is labeled. The RNA or nucleic acid derived therefrom is then hybridized to a filter containing oligonucleotides derived from one or more marker genes, wherein the oligonucleotides are placed upon the filter at discrete, easily-identifiable locations. Hybridization, or lack thereof, of the labeled RNA to the filter-bound oligonucleotides is determined visually or by densitometer. Polynucleotides can be labeled using a radiolabel or a fluorescent (i.e., visible) label.

These examples are not intended to be limiting; other methods of determining RNA abundance are known in the art.

Finally, expression of marker genes in a number of tissue specimens may be characterized using a "tissue array" (Kononen, et al., Nat. Med, 1998, 4(7):844-847). In a tissue array, multiple tissue samples are assessed on the same microarray. The arrays allow in situ detection of RNA and protein levels; consecutive sections allow the analysis of multiple samples simultaneously.

Microarrays

In some embodiments, polynucleotide microarrays are used to measure expression so that the expression status of each of the markers in one or more of the inventive gene sets, described herein, is assessed simultaneously. The microarrays of the invention preferably comprise at least 2, 3, 4, 5 or more of markers, or all of the markers, or any combination of markers, identified as classification-informative within a subject subset. The actual number of informative markers the microarray comprises will vary depending upon the particular condition of interest, the number of markers identified, and, optionally, the number of informative markers found to result in the least Type I error, Type II error, or Type I and Type II error in determination of an endpoint phenotype. As used herein, "Type I error" means a false positive and "Type II error" means a false negative; in the example of predicting a patient's therapeutic response to exposure to a CDK inhibitor, Type I error is the mischaracterization of an individual with a therapeutic response to a CDK inhibitor as being a non-responsive to CDK inhibitor treatment, and Type II error is the mischaracterization of an individual with no response to CDK inhibitor treatment as having a therapeutic response.

In specific embodiments, the invention provides polynucleotide arrays in which the markers identified for a particular subject subset comprise at least 50%, 60%, 70%, 80%, 85%, 90%, 95% or 98% of the probes on said array. In another specific embodiment, the microarray comprises a plurality of probes, wherein said plurality of probes comprise probes complementary and hybridizable to at least 75% of the CDK inhibitor exposure/prediction-informative markers identified for a particular patient subset. Microarrays of the invention, of course, may comprise probes complementary to and which are capable of hybridizing to CDK inhibitor prediction/evaluation-informative markers for a plurality of the subject subsets, or for each subject subset, identified for a particular condition. In furtherance thereof, a microarray of the invention comprises a plurality of probes complementary to and which hybridize to at least 75% of the CDK inhibitor prediction/evaluation-informative markers identified for each subject subset identified for the condition of interest, and wherein said probes, in total, are at least 50% of the probes on said microarray.

In yet another specific embodiment, the microarray is a commercially-available cDNA microarray that comprises at least two markers identified by the methods described herein. Preferably, a commercially-available

cDNA microarray comprises all of the markers identified by the methods described herein as being informative for a patient subset for a particular condition. However, such a microarray may comprise at least 1, 2, 3, 4 or 5 of such markers, up to the maximum number of markers identified.

Any of the microarrays described herein may be provided in a sealed container in a kit.

In other embodiments, the array comprises a plurality of probes derived from markers listed in any of Tables 1 in combination with a plurality of other probes, derived from markers not listed in any of Tables 1, that are identified as informative for the prediction of sensitivity to a CDK inhibitor, evaluation of therapeutic response, etc.

Polynucleotides Used to Measure the Products of the Biomarkers of the Invention

Polynucleotides capable of specifically or selectively binding to the mRNA transcripts encoding the polypeptide biomarkers of the invention are also contemplated. For example: oligonucleotides, cDNA, DNA, RNA, PCR products, synthetic DNA, synthetic RNA, or other combinations of naturally occurring or modified nucleotides which specifically and/or selectively hybridize to one or more of the RNA products of the biomarker of the invention are useful in accordance with the invention.

In a preferred embodiment, the oligonucleotides, cDNA, DNA, RNA, PCR products, synthetic DNA, synthetic RNA, or other combinations of naturally occurring or modified nucleotides oligonucleotides which both specifically and selectively hybridize to one or more of the RNA products of the biomarker of the invention are used.

To determine the (increased or decreased) expression levels of genes in the practice of the present invention, any method known in the art may be utilized. In one embodiment of the invention, expression based on detection of RNA which hybridizes to the genes identified and disclosed herein is used. This is readily performed by any RNA detection or amplification methods known or recognized as equivalent in the art such as, but not limited to, reverse transcription-PCR, and methods to detect the presence, or absence, of RNA stabilizing or destabilizing sequences.

Alternatively, expression based on detection of DNA status may be used. Detection of the DNA of an identified gene as may be used for genes that have increased expression in correlation with a particular outcome. This may be readily performed by PCR based methods known in the art, including, but not limited to, Q-PCR. Conversely, detection of the DNA of an identified gene as amplified may be used for genes that have increased expression in correlation with a particular treatment outcome. This may be readily performed by PCR based, fluorescent in situ hybridization (FISH) and chromosome in situ hybridization (CISH) methods known in the art.

Techniques to Measure the RNA Products of the Biomarkers of the Invention

Real-time PCR

In practice, a gene expression-based expression assay based on a small number of genes, i.e., about 1 to 3000 genes can be performed with relatively little effort using existing quantitative real-time PCR technology familiar to clinical laboratories. Quantitative real-time PCR measures PCR product accumulation through a dual-labeled fluorigenic probe. A variety of normalization methods may be used, such as an internal competitor for each target sequence, a normalization gene contained within the sample, or a housekeeping gene. Sufficient RNA for real time PCR can be isolated from low milligram quantities from a subject. Quantitative thermal cyclers may now be used with microfluidics cards preloaded with reagents making routine clinical use of multigene expression-based assays a realistic goal.

The gene markers of the various inventive genesets or a subset of genes selected from the inventive genesets, which are assayed according to the present invention are typically in the form of total RNA or mRNA or reverse transcribed total RNA or mRNA. General methods for total and mRNA extraction are well known in the art and are disclosed in standard textbooks of molecular biology, including Ausubel et al., Current Protocols of Molecular Biology, John Wiley and Sons (1997). RNA isolation can also be performed using purification kit, buffer set and protease from commercial manufacturers, such as Qiagen (Valencia, CA) and Ambion (Austin, TX), according to the manufacturer's instructions.

TAQman quantitative real-time PCR can be performed using commercially available PCR reagents (Applied Biosystems, Foster City, CA) and equipment, such as ABI Prism 7900HT Sequence Detection System (Applied Biosystems) according the manufacturer's instructions. The system consists of a thermocycler, laser, charge-coupled device (CCD), camera, and computer. The system amplifies samples in a 96-well or 384-well format on a thermocycler. During amplification, laser-induced fluorescent signal is collected in real-time through fiber-optics cables for all 96 wells, and detected at the CCD. The system includes software for running the instrument and for analyzing the data.

Based upon the marker gene sets identified in the present invention, a real-time PCR TAQman assay can be used to make gene expression measurements and perform the classification methods described herein. As is apparent to a person of skill in the art, a wide variety of oligonucleotide primers and probes that are complementary to or hybridize to the markers of the invention may be selected based upon the marker transcript sequences set forth in the Sequence Listing.

Array Hybridization

The polynucleotide used to measure the RNA products of the invention can be used as nucleic acid members stably associated with a support to comprise an array according to one aspect of the invention. The length of a nucleic acid member can range from 8 to 1000 nucleotides in length and are chosen so as to be specific for the RNA products of the biomarkers of the invention. In one embodiment, these members are selective for the RNA products of the invention. The nucleic acid members may be single or double stranded, and/or may be oligonucleotides or PCR fragments amplified from cDNA. Preferably oligonucleotides are approximately 20-30 nucleotides in length. ESTs are preferably 100 to 600 nucleotides in length. It will be understood to a person skilled in the art that one can utilize portions of the expressed regions of the biomarkers of the invention as a probe on the array. More particularly oligonucleotides complementary to the genes of the invention and cDNA or ESTs derived from the genes of the invention are useful. For oligonucleotide based arrays, the selection of oligonucleotides corresponding to the gene of interest which are useful as probes is well understood in the art. More particularly it is important to choose regions which will permit hybridization to the target nucleic acids. Factors such as the Tm of the oligonucleotide, the percent GC content, the degree of secondary structure and the length of nucleic acid are important factors. See for example U.S. Pat. No. 6,551,784.

Construction of a Nucleic Acid Array

In the proposed methods, an array of nucleic acid members stably associated with the surface of a substantially support is contacted with a sample comprising target nucleic acids under hybridization conditions sufficient to produce a hybridization pattern of complementary nucleic acid members/target complexes in which one or more complementary nucleic acid members at unique positions on the array specifically hybridize to target nucleic acids. The identity of target nucleic acids which hybridize can be determined with reference to location of nucleic acid members on the array.

The nucleic acid members may be produced using established techniques such as polymerase chain reaction (PCR) and reverse transcription (RT). These methods are similar to those currently known in the art (see, PCR Strategies, Michael A. Innis (Editor), et al., (1995) and PCR: Introduction to Biotechniques Series, C. R. Newton, A. Graham (1997)). Amplified nucleic acids are purified by methods well known in the art (e.g., column purification or alcohol precipitation). A nucleic acid is considered pure when it has been isolated so as to be substantially free of primers and incomplete products produced during the synthesis of the desired nucleic acid. Preferably, a purified nucleic acid will also be substantially free of contaminants which may hinder or otherwise mask the specific binding activity of the molecule.

An array, according to one aspect of the invention, comprises a plurality of nucleic acids attached to one surface of a support at a density exceeding 20 different nucleic acids/cm$^2$, wherein each of the nucleic acids is attached to the surface of the support in a non-identical pre-selected region (e.g. a microarray). Each associated sample on the array comprises a nucleic acid composition, of known identity, usually of known sequence, as described in greater detail below. Any conceivable substrate may be employed in the invention.

In one embodiment, the nucleic acid attached to the surface of the support is DNA. In one embodiment, the nucleic acid attached to the surface of the support is cDNA or RNA. In another embodiment, the nucleic acid attached to the surface of the support is cDNA synthesized by polymerase chain reaction (PCR). Usually, a nucleic acid member in the array, according to the invention, is at least 10, 25, 50, 60 nucleotides in length. In one embodiment, a nucleic acid member is at least 150 nucleotides in length. Preferably, a nucleic acid member is less than 1000 nucleotides in length. More preferably, a nucleic acid member is less than 500 nucleotides in length.

In the arrays of the invention, the nucleic acid compositions are stably associated with the surface of a support, where the support may be a flexible or rigid support. By "stably associated" is meant that each nucleic acid member maintains a unique position relative to the support under hybridization and washing conditions. As such, the samples are non-covalently or covalently stably associated with the support surface. Examples of non-covalent association include non-specific adsorption, binding based on electrostatic interactions (e.g., ion pair interactions), hydrophobic interactions, hydrogen bonding interactions, specific binding through a specific binding pair member covalently attached to the support surface, and the like. Examples of covalent binding include covalent bonds formed between the nucleic acids and a functional group present on the surface of the rigid support (e.g., --OH), where the functional group may be naturally occurring or present as a member of an introduced linking group, as described in greater detail below.

The amount of nucleic acid present in each composition will be sufficient to provide for adequate hybridization and detection of target nucleic acid sequences during the assay in which the array is employed. Generally, the amount of each nucleic acid member stably associated with the support of the array is at least about 0.001 ng, preferably at least about 0.02 ng and more preferably at least about 0.05 ng, where the amount may be as high as 1000 ng or higher, but will usually not exceed about 20 ng. Where the nucleic acid member is "spotted" onto the support in a spot comprising an overall circular dimension, the diameter of the "spot" will generally range from about 10 to 5,000 $\mu$m, usually from about 20 to 2,000 $\mu$m and more usually from about 100 to 200 $\mu$m.

Control nucleic acid members may be present on the array including nucleic acid members comprising oligonucleotides or nucleic acids corresponding to genomic DNA, housekeeping genes, vector sequences, plant nucleic acid sequence, negative and positive control genes, and the like. Control nucleic acid members are calibrating or control genes whose function is not to tell whether a particular "key" gene of interest is expressed, but rather to provide other useful information, such as background or basal level of expression.

Other control nucleic acids are spotted on the array and used as target expression control nucleic acids and mismatch control nucleotides to monitor non-specific binding or cross-hybridization to a nucleic acid in the sample other than the target to which the probe is directed. Mismatch probes thus indicate whether a hybridization is specific or not. For example, if the target is present, the perfectly matched probes should be consistently brighter than the mismatched probes. In addition, if all control mismatches are present, the mismatch probes are used to detect a mutation.

Numerous methods may be used for attachment of the nucleic acid members of the invention to the substrate (a process referred to as "spotting"). For example, nucleic acids are attached using the techniques of, for example U.S. Pat. No. 5,807,522, which is incorporated herein by reference, for teaching methods of polymer attachment. Alternatively, spotting may be carried out using contact printing technology as is known in the art.

The measuring of the expression of the RNA product of the invention can be done by using those polynucleotides which are specific and/or selective for the RNA products of the invention to quantitate the expression of the RNA product. In a specific embodiment of the invention, the polynucleotides which are specific and/or selective for the RNA products are probes or primers. In one embodiment, these polynucleotides are in the form of nucleic acid probes which can be spotted onto an array to measure RNA from the sample of an individual to be measured. In another embodiment, commercial arrays can be used to measure the expression of the RNA product. In yet another embodiment, the polynucleotides which are specific and/or selective for the RNA products of the invention are used in the form of probes and primers in techniques such as quantitative real-time RT PCR, using for example SYBR®Green, or using TaqMan® or Molecular Beacon techniques, where the polynucleotides used are used in the form of a forward primer, a reverse primer, a TaqMan labeled probe or a Molecular Beacon labeled probe. Where only one or two genes are to be analyzed, the nucleic acid derived from the sample cell(s) may be preferentially amplified by use of appropriate primers such that only the genes to be analyzed are amplified to reduce background signals from other genes expressed in the breast cell. Alternatively, and where multiple genes are to be analyzed or where very few cells (or one cell) is used, the nucleic acid from the sample may be globally amplified before hybridization to the immobilized polynucleotides. Of course RNA, or the cDNA counterpart thereof may be directly labeled and used, without amplification, by methods known in the art.

Use of a Microarray

A "microarray" is a linear or two- dimensional array of preferably discrete regions, each having a defined area, formed on the surface of a solid support such as, but not limited to, glass, plastic, or synthetic membrane. The density of the discrete regions on a microarray is determined by the total numbers of immobilized polynucleotides to be detected on the surface of a single solid phase support, preferably at least about 50/$cm^2$, more preferably at least about 100/$cm^2$, even more preferably at least about 500/$cm^2$, but preferably below about 1,000/$cm^2$. Preferably, the arrays contain less than about 500, about 1000, about 1500, about 2000, about 2500, or about 3000 immobilized polynucleotides in total. As used herein, a DNA microarray is an array of oligonucleotides or polynucleotides placed on a chip or other surfaces used to hybridize to amplified or cloned polynucleotides from a sample. Since the position of each particular group of primers in the array is known, the identities of a sample polynucleotides can be determined based on their binding to a particular position in the microarray.

Determining gene expression levels may be accomplished utilizing microarrays. Generally, the following steps may be involved: (a) obtaining an mRNA sample from a subject and preparing labeled nucleic acids therefrom (the "target nucleic acids" or "targets"); (b) contacting the target nucleic acids with an array under conditions sufficient for the target nucleic acids to bind to the corresponding probes on the array, for example, by hybridization or specific binding; (c) optional removal of unbound targets from the array; (d) detecting the bound targets, and (e) analyzing the results, for example, using computer based analysis methods. As used herein, "nucleic acid probes" or "probes" are nucleic acids attached to the array, whereas "target nucleic acids" are nucleic acids that are hybridized to the array.

Nucleic acid specimens may be obtained from a subject to be tested using either "invasive" or "non-invasive" sampling means. A sampling means is said to be "invasive" if it involves the collection of nucleic acids from within the skin or organs of an animal (including murine, human, ovine, equine, bovine, porcine, canine, or feline animal). Examples of invasive methods include, for example, blood collection, semen collection, needle biopsy, pleural aspiration, umbilical cord biopsy. Examples of such methods are discussed by Kim, et al, J. Virol., 1992, 66:3879-3882, Biswas, et al., Ann. NY Acad. Sci., 1990, 590:582-583, and Biswas, et al., J. Clin. Microbiol., 1991, 29:2228-2233.

In contrast, a "non-invasive" sampling means is one in which the nucleic acid molecules are recovered from an internal or external surface of the animal. Examples of "non-invasive" sampling means include, but are not limited to, "swabbing," or the collection of tears, saliva, urine, or fecal material.

In one embodiment of the present disclosure one or more cells from the subject to be tested are obtained and RNA is isolated from the cells. In one embodiment, a sample of cells is obtained from the subject. It is also possible to obtain a cell sample from a subject, and then to enrich the sample for a desired cell type. For example, cells may be isolated from other cells using a variety of techniques, such as isolation with an antibody binding to an epitope on the cell surface of the desired cell type. Where the desired cells are in a solid tissue, particular cells may be dissected, for example, by microdissection or by laser capture microdissection (LCM) (see, Bonner, et al., Science, 1997, 278:1481; Emmert-Buck, et al., Science, 1996, 274:998; Fend, et al., Am. J. Path., 1999, 154:61; and Murakami, et al., Kidney Hit., 2000, 58:1346).

RNA may be extracted from tissue or cell samples by a variety of methods, for example, guanidium thiocyanate lysis followed by CsCl centrifugation (Chirgwin, et al., Biochemistry, 1979, 18:5294-5299). RNA from single cells may be obtained as described in methods for preparing cDNA libraries from single cells (see, Dulac, Curr. Top. Dev. Biol., 1998, 36:245; Jena, et al., J. Immunol. Methods, 1996, 190:199).

The RNA sample can be further enriched for a particular species. In one embodiment, for example, poly(A)+RNA may be isolated from an RNA sample. In another embodiment, the RNA population may be enriched for sequences of interest by primer-specific cDNA synthesis, or multiple rounds of linear amplification based on cDNA synthesis and template- directed in vitro transcription (see, Wang, et al., Proc. Natl. Acad. Sci. USA, 1989, 86:9717; Dulac, *et al., supra;* Jena, *et al., supra*). In addition, the population of RNA, enriched or not in particular species or sequences, may be further amplified by a variety of amplification methods including, for example, PCR; ligase chain reaction (LCR) (see, Wu and Wallace, Genomics, 1989, 4:560; Landegren, et al., Science, 1988, 241:1077); self- sustained sequence replication (SSR) (see, Guatelli, et al., Proc. Natl. Acad. Sci. USA, 1990, 87:1874); nucleic acid based sequence amplification (NASBA) and transcription amplification (see, Kwoh, et al., Proc. Natl. Acad. Sci. USA, 1989, 86:1173). Methods for PCR technology are well known in the art (see, PCR Technology: Principles and Applications for DNA Amplification, ed. H. A. Erlich, Freeman Press, N.Y., N.Y., 1992; PCR Protocols: A Guide to Methods and Applications, eds. Innis, et al., Academic Press, San Diego, Calif., 1990; Mattila, et al., Nucleic Acids Res., 19:4967, 1991; Eckert, et al., PCR Methods and Applications, 1991,1:17; PCR, eds. McPherson et al., IRL Press, Oxford); and U.S. Pat. No. 4,683,202). Methods of amplification are described, for example, by Ohyama, et al, BioTechniques, 2000, 29:530; Luo, et al., Nat. Med., 1999, 5:117; Hegde, et al., BioTechniques, 2000, 29:548; Kacharmina, et al., Meth. Enzymol., 1999, 303:3; Livesey, et al., Curr. Biol., 2000, 10:301; Spirin, et al., Invest. Ophtalmol. Vis. Sci., 1999, 40:3108; and Sakai, et al., Anal. Biochem., 2000, 287:32). RNA amplification and cDNA synthesis may also be conducted in cells in situ (see, Eberwine, et al., Proc. Natl. Acad. Sci. USA, 1992, 89:3010.

In yet another embodiment of the invention, all or part of a disclosed marker sequence may be amplified and detected by methods such as the polymerase chain reaction (PCR) and variations thereof, such as, but not limited to, quantitative PCR (Q-PCR), reverse transcription PCR (RT-PCR), and real-time PCR, optionally real-time RT-PCR. Such methods would utilize one or two primers that are complementary to portions of a disclosed sequence, where the primers are used to prime nucleic acid synthesis.

The newly synthesized nucleic acids are optionally labeled and may be detected directly or by hybridization to a polynucleotide of the invention.

The nucleic acid molecules may be labeled to permit detection of hybridization of the nucleic acid molecules to a microarray. That is, the probe may comprise a member of a signal producing system and thus, is detectable, either directly or through combined action with one or more additional members of a signal producing system. For example, the nucleic acids may be labeled with a fluorescently labeled dNTP (see, Kricka, Nonisotopic DNA Probe Techniques, Academic Press San Diego, CA, 1992), biotinylated dNTPs or rNTP followed by addition of labeled streptavidin, chemiluminescent labels, or isotopes. Another example of labels include "molecular beacons" as described in Tyagi and Kramer, Nature Biotech., 1996, 14:303. The newly synthesized nucleic acids may be contacted with polynucleotides (containing sequences) of the invention under conditions which allow for their hybridization. Hybridization may be also determined, for example, by plasmon resonance (see, Thiel, et al., Anal. Chem., 1997, 69:4948).

In one embodiment, a plurality, e.g., two sets of target nucleic acids are labeled and used in one hybridization reaction ("multiplex" analysis). For example, one set of nucleic acids may correspond to RNA from one cell and another set of nucleic acids may correspond to RNA from another cell. The plurality of sets of nucleic acids may be labeled with different labels, for example, different fluorescent labels (e.g., fluorescein and rhodamine) which have distinct emission spectra so that they can be distinguished. The sets may then be mixed and hybridized simultaneously to one microarray (see, Shena, et al., Science, 1995, 270:467-470).

A number of different microarray configurations and methods for their production are known to those of skill in the art and are disclosed in U.S. Pat. Nos: 5,242,974; 5,384,261; 5,405,783; 5,412, 087; 5,424,186; 5,429, 807; 5,436,327; 5,445,934; 5,556,752; 5,405,783; 5, 412,087; 5,424,186; 5, 429,807; 5,436,327; 5,472,672; 5,527,681; 5,529, 756; 5,545,531; 5,554,501;

5,561,071; 5,571,639; 5,593,839; 5,624,711; 5, 700,637; 5,744,305; 5,770, 456; 5,770,722; 5,837,832; 5,856,101; 5,874, 219; 5,885,837; 5,919,523; 6, 022,963; 6,077,674; and 6,156,501; Shena, et al., Tibtech, 1998, 16:301; Duggan, et al., Nat.Genet., 1999, 21:10; Bowtell, et al., Nat. Genet., 1999, 21:25; Lipshutz, et al., Nature Genet., 1999, 21:20-24; Blanchard, et al., Biosensors and Bioelectronics, 11:687-690, 1996; Maskos, et al., Nucleic Acids Res., 1993, 21:4663-4669; Hughes, et al., Nat. Biotechol., 2001, 19:342; the disclosures of which are herein incorporated by reference. Patents describing methods of using arrays in various applications include: U.S. Pat. Nos.: 5,143,854; 5,288, 644; 5,324,633; 5, 432,049; 5,470,710; 5,492,806; 5,503,980; 5,510,270; 5, 525,464; 5,547,839; 5,580,732; 5,661,028; 5,848,659; and 5,874,219; the disclosures of which are herein incorporated by reference.

In one embodiment, an array of oligonucleotides may be synthesized on a solid support. Exemplary solid supports include glass, plastics, polymers, metals, metalloids, ceramics, organics, etc. Using chip masking technologies and photoprotective chemistry, it is possible to generate ordered arrays of nucleic acid probes. These arrays, which are known, for example, as "DNA chips" or very large scale immobilized polymer arrays ("VLSIPS®" arrays), may include millions of defined probe regions on a substrate having an area of about 1 $cm^2$ to several $cm^2$, thereby incorporating from a few to millions of probes (see, U.S. Pat. No. 5,631,734).

To compare expression levels, labeled nucleic acids may be contacted with the array under conditions sufficient for binding between the target nucleic acid and the probe on the array. In one embodiment, the hybridization conditions may be selected to provide for the desired level of hybridization specificity; that is, conditions sufficient for hybridization to occur between the labeled nucleic acids and probes on the microarray.

Hybridization may be carried out in conditions permitting essentially specific hybridization. The length and GC content of the nucleic acid will determine the thermal melting point and thus, the hybridization conditions necessary for obtaining specific hybridization of the probe to the target nucleic acid. These factors are well known to a person of skill in the art, and may also be tested in assays. An extensive guide to nucleic acid hybridization may be found in Tijssen, et al., Laboratory Techniques in Biochemistry and Molecular Biology, Vol. 24: Hybridization With Nucleic Acid Probes, P. Tijssen, ed., Elsevier, N.Y., 1993.

The methods described above will result in the production of hybridization patterns of labeled target nucleic acids on the array surface. The resultant hybridization patterns of labeled nucleic acids may be visualized or detected in a variety of ways, with the particular manner of detection selected based on the particular label of the target nucleic acid. Representative detection means include scintillation counting, autoradiography, fluorescence measurement, calorimetric measurement, light emission measurement, light scattering, and the like.

One such method of detection utilizes an array scanner that is commercially available (Affymetrix, Santa Clara, Calif.), for example, the 417® Arrayer, the 418® Array Scanner, or the Agilent GeneArray® Scanner. This scanner is controlled from a system computer with an interface and easy-to-use software tools. The output may be directly imported into or directly read by a variety of software applications. Exemplary scanning devices are described in, for example, U.S. Pat. Nos. 5,143,854 and 5,424,186.

Administration of CDK inhibitors

The predictive biomarker of the invention herein can be used to identify cancer patients predicted to be responsive to treatment with a CDK inhibitor. Patients diagnosed with cancer in which the cancer is a CDK mediated proliferative disorder or one in which the cancer cell and tumor express aberrant CDK signaling and, as such, amenable to treatment with a CDK inhibitor include, but are not limited to, acute myelogenous leukemia (AML), chronic myelogenous leukemia (CML), acute lymphocytic leukemia (ALL), and chronic lymphocytic leukemia, Kaposi's sarcoma; breast cancers; bone cancers, brain cancers, cancers of the head and neck, gallbladder and bile duct cancers, cancers of the retina, cancers of the esophagus, gastric cancers, multiple myeloma, ovarian cancer, uterine cancer, thyroid cancer, testicular cancer, endometrial cancer, melanoma, colorectal cancer, bladder cancer, prostate cancer, lung cancer, pancreatic cancer, sarcomas, Wilms' tumor, cervical cancer, skin cancers, nasopharyngeal carcinoma, liposarcoma, epithelial carcinoma, renal cell carcinoma, gallbladder adenocarcinoma, parotid adenocarcinoma, and endometrial sarcoma.

The CDK inhibitor can be administered by any known administration method known to a person skilled in the art. Examples of routes of administration include but are not limited to oral, parenteral, intraperitoneal, intravenous, intraarterial, transdermal, sublingual, intramuscular, rectal, transbuccal, intranasal, liposomal, via inhalation, vaginal, intraoccular, via local delivery by catheter or stent, subcutaneous, intraadiposal, intraarticular, intrathecal, or in a slow release dosage form.

The CDK inhibitors or a pharmaceutically acceptable salt or hydrate thereof, can be administered in accordance with any dose and dosing schedule that, achieves a dose effective to treat cancer. For example, CDK inhibitors can be administered in a total daily dose of up to 1000 mg, preferably orally, once, twice or three times daily, continuously (every day) or intermittently (e.g., 3-5 days a week).

A CDK inhibitor may also be administered in combination with an anti-cancer agent, wherein the amount of CDK and the amount of the anti-cancer agent together comprise a therapeutically effective amount. The combination therapy can provide a therapeutic advantage in view of the differential toxicity associated with the two treatment modalities. For example, treatment with CDK inhibitors can lead to a particular toxicity that is not seen with the anti-cancer agent, and vice versa. As such, this differential toxicity can permit each treatment to be administered at a dose at which said toxicities do not exist or are minimal, such that together the combination therapy provides a therapeutic dose while avoiding the toxicities of each of the constituents of the combination agents. Furthermore, when the therapeutic effects achieved as a result of the combination treatment are enhanced or synergistic, for example, significantly better than additive therapeutic effects, the doses of each of the agents can be reduced even further, thus lowering the associated toxicities to an even greater extent.

CDK inhibitor can be combined with chemotherapy and radiotherapy. CDK inhibitor is also combined with an anti-cancer agent, but is preferably combined with a DNA damaging agents. Examples of such anti-cancer agent used in a combination treatment with CDK inhibitors are for example, but not limited to, gemcitabine, cisplatin, carboplatin, 5-fluorouracil, pemetrexed, doxorubicin, camptothecin and mitomycin. A CDK inhibitor may be administered in a pharmaceutical composition, preferably suitable for oral administration. A CDK inhibitor may be administered orally in a gelating capsule, which can comprise excipients such as microcrystalline cellulose, croscarmellose sodium and magnesium stearate.

The CDK inhibitors can be administered in a total daily dose that may vary from patient to patient, and may be administered at varying dosage schedules. Suitable dosages are total daily dosage of between about 25-4000 mg/$m^2$ administered orally once-daily, twice-daily or three times-daily, continuous (every day) or intermittently (e.g. 3-5 days a week). The compositions may also be administered in cycles, with rest periods in between the cycles (e.g. treatment for two to eight weeks with a rest period of up to a week between treatments). Other suitable treatment combinations and dosing regiments are set forth in WO 2007/126122, WO2007/126128, and WO 2008/133866.

One skilled in the art would recognize and appreciate that any one or more of the specific dosages and dosage schedules listed for a CDK inhibitor herein may also be applicable for use in combination with one or more of the anti-cancer agents. One skilled in the art would also recognize and appreciate that the specific dosage and dosage schedule of the anti-cancer agent can vary and that the optimal dose, dosing schedule, and route of administration will be determined based upon the specific anti-cancer agent that is being used in combination.

## EXAMPLES

### Example 1

Materials and Methods

A. Reagents

SCH 727965 (Dinaciclib) was dissolved in dimethyl sulfoxide (DMSO; Sigma-Aldrich, St. Louis, MO) at 10 mmol/L and aliquots were stored at -80°C.

B. Cell culture

Cell lines were obtained from the American Type Culture Collection, the European Collection of Cell Cultures, or the Deutsche Sammlung von Mikroorganismen und Zellkulturen. Cell lines were cultured using standard techniques in RPMI 1640, DMEM, F-12K or L-15 medium supplemented with 10% FBS (Invitrogen™, Life Technologies™, Grand Island, NY) and/or HEPES, L-glutamine, glucose or sodium bicarbonate.

C. Western blotting and band quantification

Cells were seeded in 10-cm cell culture dishes, cultured to 60-90% confluency, and treated with 50 or 100 nM SCH 727965 (Dinaciclib). DMSO was used as a solvent-only negative control. After specified treatment times, culture medium was removed, cells were washed with 4°C chilled Dulbecco's phosphate-buffered saline (DPBS) and lysed in ice-cold radioimmunoprecipitation assay (RIPA) buffer (Cell Signaling Technology®, Beverly, MA) containing protease inhibitor (Roche Diagnostics (Applied Biosciences, Indianapolis, IN), complete mini EDTA-free tablet) and phosphatase inhibitor (Roche Diagnostics (Applied Biosciences, Indianapolis, IN), PhosSTOP tablet) cocktails. Cell lysates were collected using a cell lifter (Costar®, Sigma Aldrich, St. Louis, MO), transferred to microfuge tubes, briefly sonicated, and non-soluble material was removed by microfuge at 12 kpm, 4°C, 10 min. In cases of SCH 727965 (Dinaciclib)-induced cell detachment, the culture media and DPBS wash were collected and cells were collected by centrifugation and pooled with the plate RIPA lysate.

Equal amounts or proportional dilutions of proteins were separated electrophoretically on NuPAGE 4 to 12% Bis-Tris polyacrylamide gels (Invitrogen™, Life Technologies™, Grand Island, NY). Gels were transferred to PVDF membranes (Invitrogen™, Life Technologies™, Grand Island, NY) and blocked with StartingBlock buffer (Thermo Fisher Scientific, Waltham, MA).

Primary antibodies (Cell Signaling Technology®, Beverly, MA) directed against the following proteins were used: Cleaved PARP (9541), MCL-1 (5453), MYC (5605), alpha-Tubulin (3873), BCL-xL (2765) and BCL-2 (2870). Antibodies against RNA pol II (8WG16) and RNA pol II phosphoserine2 (H5) were from Covance,

Princeton, NJ (MPY-127R). Membranes were probed with primary and secondary antibodies in StartingBlock and washed with TBS-Tween-20 (Thermo Fisher Scientific, Waltham, MA). The secondary HRP-linked antibodies (Cell Signaling Technology®, Beverly, MA) were detected using SuperSignal West Pico or Femto chemiluminescent substrate (Thermo Fisher Scientific, Waltham, MA) with autoradiography film (Kodak, BioMax MR, Sigma Aldrich, St. Louis) and bands were quantified using ImageQuant TL 7.0 (GE Healthcare Life Sciences).

D. RNA quantification

RNA quantification as shown in Figures 6A and 6B was carried out as follows. Cells were seeded in 10-cm cell culture dishes, cultured to 80-90% confluency and treated with 100 nM dinaciclib. DMSO was used as a solvent-only negative control. After specified treatment times, culture medium was removed, cells were washed with room-temperature DPBS, dissociated from plates using trypsin (Invitrogen) and diluted with fresh growth medium. 1.5 x$10^5$ cells in 0.4 ml growth medium were collected in 1.5 ml microfuge tubes. Working Lysis Mixture was prepared and cells were lysed according to instructions provided in the QuantiGene Sample Processing Kit (Affymetrix, Panomics, Santa Clara, CA). RNA levels were quantified by QuantiGene Plex 2.0 Assay (Affymetrix, Panomics, Santa Clara, CA) using a magnetic plate washer according to the User Manual instructions. Target-specific Probe Set 11837, Human 311836-105 was used to quantify transcripts expressed from TUBA1B, MCL1, BCL2, GAPDH, CCNE1, BCL2L1 and BCL2L2.

E. Cell viability and caspase-3/7 assays

Cells were seeded in 96-well plates at sub-confluent densities. The following day, cells were treated for specified times with 25, 50 or 100 nM dinaciclib. DMSO was used as a solvent-only negative control. Cell viability was determined from a minimum of three cell-containing wells per treatment on a minimum of two independent plates using CellTiter-Glo Assay System (Promega, Madison, WI). Apoptotic induction was determined from a minimum of three cell-containing wells per treatment using Caspase-Glo 3/7 Assay System (Promega, Madison, WI). Changes in cell viability or intracellular caspase-3/7 activity were calculated as a percentage relative to the DMSO control. Statistical analysis was performed using GraphPad Prism (GraphPad Software, La Jolla, CA).

F. MCL-1:BCL-xL mRNA ratio

Cell line MCL-1 and BCL-xL expression levels ($log_{10}$) were obtained from the Cancer Cell Line Encyclopedia (CCLE).

Example 2

Sensitivity of cancer cell lines to short-term treatment with a CDK inhibitor

Twenty-three lung, breast, ovarian, colorectal, and prostate cell lines, with varying gene expression levels for the MCL-1:BCL-xL ratio, were treated with the CDK inhibitor, SCH 727965 (Dinaciclib), for 18 hours as shown in Materials and Methods, Example 1. The viability of the cell lines was measured by a cytotoxic assay. As shown in Figure 5B, cell viability among the cell lines treated for 18 hours with SCH 727965 (Dinaciclib) was variable, ranging from 23% to 102%, relative to a DMSO-negative control treatment. As shown graphically in Figure 5B, a significant correlation was observed between the cellular viability and the gene expression level of the MCL-1:BCL-xL ratio.

Example 3

Induction of apoptosis in cancer cell lines to short-term treatment with a CDK inhibitor

Twenty-seven lung, breast, ovarian, colorectal, and prostate cell lines, with varying gene expression levels for the MCL-1:BCL-xL ratio, were treated with the CDK inhibitor, SCH 727965 (Dinaciclib) for 8 hours. The level of apoptotic induction was determined by measurement of the PARP cleavage product by Western blot as described in Example 1. Results of the 8 hour treatment with SCH 727965 (Dinaciclib) are shown in Figures 8 and 9A, in which apoptotic induction among the treated cell lines treated was variable. As shown graphically in Figure 9A, a significant correlation was observed between apoptotic induction, as measured by PARP cleavage, and the gene expression level for the MCL-1:BCL-xL ratio.

Example 4

CDK inhibitor sensitivity correlation to high MCL-1:BCL-xL mRNA expression ratio

High MCL-1:BCL-xl mRNA expression ratio correlates with sensitivity to a CDK inhibitor, as shown in Figure 10 by the correlation coefficients and p-values for the percentage of cells remaining viable relative to the level of gene expression for the MCL-1 :BCL-xL ratio for 387 heme (leukemia and lymphoma) and solid tumor cell lines (as a measure of apoptosis) after treatment for 24 hours with the CDK inhibitor, SCH 727965 (Dinaciclib). The measurement of cell viability and mRNA expression for the ratio was determined as described in the Materials and Methods, Example 1. As shown, the correlation coefficient (r) was -0.41 (p=5e-17), indicative of the statistical correlation of these values.

Example 5

Induction of apoptosis in high MCL-1:BCL-xL ratio solid tumor xenografts

Dinaciclib was formulated in 20% hydroxypropyl beta-cyclodextrin (HPBCD) in de-ionized water (vehicle). Xenograft tumor studies were conducted in house by Applicants (NCI-H23 and COLO-320DM) and by Piedmont Research Center (Morrisville, NC) (A2780, 22Rv1, SW480, JIMT-1, MDA-MB-231 and PC3). All animal studies were performed according to guidelines established by the Institutional Animal Care and Use Committee of each institution.

Xenograft studies were conducted by implantation of 1 x $10^7$ A2780 cells in PBS, 5 x $10^6$ MDA-MB-231 cells in PBS, about 1 $mm^3$ SW480 tumor fragments, or about 1 $mm^3$ PC3 tumor fragments in the flanks of female athymic nude mice (Crl:NU(NCr)-Foxnlnu, Charles River Laboratories); 5 x $10^6$ NCI-H23 in 50% Matrigel (BD Biosciences), 5 x $10^6$ COLO-320DM cells in 50% Matrigel, or 1 x $10^7$ JIMT-1 cells in 50% Matrigel in the flanks of female SCID mice (Fox Chase SCID, C.B-17/Icr-Prkdcscid, Charles River Laboratories); and 1 x $10^7$ 22Rv1 cells in 50% Matrigel in the flanks of male athymic nude mice (nu/nu, Harlan). Tumor samples were collected 6 hours post-dosing from animals in two sampling groups (n =5), that received one dose of vehicle or 40 mg/kg SCH 727965 (Dinaciclib) when the mean tumor volume reached 300 to 400 $mm^3$. Figure 11 is the analysis of two representative tumor lysates from the 5 tumors collected per treatment cohort. Each tumor was divided into three parts. Two parts were snap frozen in liquid nitrogen and stored at -80°C. The third part was preserved in 10% neutral buffered formalin for 24 hours then stored in 70% ethanol at ambient temperature.

Whole cell lysates from one snap frozen part were prepared by disruption/homogenation at 4°C in a 2 ml conical-end microfuge tube containing two stainless steel beads (5/32", grade 25, Ball Supply Corp) and 0.4 ml lysis buffer [1% Triton X-100, 30 mM Tris pH 7.4, 1 mM EDTA and protease (complete, mini, EDTA-free, Roche) and phosphatase (PhosSTOP, Roche) cocktail inhibitors] using a Qiagen Lyser II, shaking at 30 Hz for 2 minutes. 200 $\mu$l of pre-chilled 3X RIPA (Cell Signaling Technology) containing protease and phosphatase cocktail inhibitors was added to the homogenate, rotated at 4°C for 10 minutes, and microcentrifuged at 12 kpm for 10 minutes at 4°C. The supernatant was removed, normalized for protein content using a BCA kit (Pierce, Rockford, IL) and stored at -80°C. For Western blots, 5 $\mu$g of lysate were subjected to SDS-PAGE except for cleaved PARP analysis, which required loading of 0.5 $\mu$g of lysate.

The rabbit polyclonal antibodies against MCL-1 (#5453) and cleaved PARP (#9541), rabbit monoclonal antibody (mAbs) against BCL-xL (#2764), and mouse mAbs against $\alpha$-tubulin (#3873) were from Cell Signaling (Beverly, MA). Secondary HRP-labeled goat anti-rabbit (#7074) and horse anti-mouse antibodies (#7076) (Cell Signaling, Beverly, MA) in conjunction with SuperSignal West Pico or Femto Chemiluminescent Substrate (Thermo Scientific) were used for protein detection.

## Claims

1. A method for identifying a cancer patient likely to be responsive to treatment with a CDK inhibitor, comprising:

   (a) measuring the value of a predictive biomarker of a CDK inhibitor in a biological sample obtained from a cancer patient;
   (b) comparing the value of the biomarker to a pre-determined reference value to determine whether the value is above or below the pre-determined reference value; and
   (c) identifying a patient likely to be responsive to treatment with a CDK inhibitor, wherein the predictive biomarker is the MCL-1:BCL-xL gene expression ratio and the responsive patient has a value above the pre-determined reference value, wherein the CDK inhibitor is (S)-(-)-(-)2-(1-{3-ethyl-7-[(1-oxy-pyridin-3-ylmethyl)] amino]pyrazolo[1,5-a]pyrimidin-5-yl}piperidin-2-yl)ethanol)(dinaciclib) or a pharmaceutically acceptable salt thereof, and the MCL-1 :BCL-xL gene expression ratio is of the mRNA level.

2. The method according to claim 1, wherein a patient having a MCL-1:BCL-xL gene expression ratio below the reference value is identified as a patient likely to be non-responsive to treatment with a CDK inhibitor.

3. The method according to claim 1, wherein the pre-determined reference value is the MCL-1:BCL-xL gene expression ratio obtained from a biological sample comprising cells from one or more patients who have not been diagnosed with cancer.

4. The method according to claim 1, wherein the pre-determined reference value is the MCL-1:BCL-xL gene expression ratio obtained from a biological sample comprising cells from one or more patients who are disease free or whose cells do not exhibit aberrant CDK signaling.

5. The method according to claim 1, wherein the cancer is selected from the group consisting of acute myelogenous leukemia (AML), chronic myelogenouse leukemia (CML), acute lymphocytic leukemia (ALL), chronic lymphocytic leukemia, Kaposi's sarcoma, breast cancer, bone cancer, brain cancer, cancer of the head and neck, gallbladder and bile duct cancers, cancers of the retina, cancers of the esophagus, gastric cancer, multiple myeloma, ovarian cancer, uterine cancer, thyroid cancer, testicular cancer, endometrial cancer, melanoma, colorectal cancer, bladder cancer, prostate cancer, lung cancer pancreatic cancer, sarcomas, Wilms' tumor, cervical cancer, skin cancer, nasopharyngeal carcinoma, liposarcoma, epithelial carcinoma, renal cell carcinoma, gallbladder adenocarcinoma, parotid adenocarcinoma, and endometrial sarcoma.

**6.** A CDK inhibitor for use in treating a cancer patient identified as likely to be responsive by the method according to any of claims 1-5, wherein the CDK inhibitor is ((S)-(-)--(-)2-(1-{3-ethyl-7-[(1-oxy-pyridin-3-ylmethyl)]amino]pyrazolo[1,5-a]pyrimidin-5-yl}piperidin-2-yl)ethanol))(dinaciclib), or a pharmaceutically acceptable salt thereof.

## Patentansprüche

**1.** Ein Verfahren zur Identifizierung eines Krebspatienten, der voraussichtlich auf eine Behandlung mit einem CDK-Inhibitor anspricht, umfassend:

(a) Messen des Werts eines prädiktiven Biomarkers eines CDK-Inhibitors in einer biologischen Probe, die einem Krebspatienten entnommen wurde,
(b) Vergleichen des Werts des Biomarkers mit einem vorgegebenen Referenzwert, um zu ermitteln, ob der Wert oberhalb oder unterhalb des vorgegebenen Referenzwerts liegt, und
(c) Identifizieren eines Patienten, der voraussichtlich auf eine Behandlung mit einem CDK-Inhibitor anspricht, wobei der prädiktive Biomarker das MCL-1:BCL-xL-Genexpressionsverhältnis ist und der ansprechende Patient einen Wert oberhalb des vorgegebenen Referenzwerts besitzt, wobei der CDK-Inhibitor (S)-(-)--(-)2-(1-{3-Ethyl-7-[(1-oxypyridin-3-ylmethyl)]amino]pyrazolo[1,5-a]-pyrimidin-5-yl}piperidin-2-yl)ethanol)(Dinaciclib) oder ein pharmazeutisch annehmbares Salz davon ist und das MCL-1:BCL-xL-Genexpressionsverhältnis vom mRNA-Level ist.

**2.** Das Verfahren gemäß Anspruch 1, wobei ein Patient mit einem MCL-1:BCL-xL-Genexpressionsverhältnis unterhalb des Referenzwerts als ein Patient identifiziert wird, der voraussichtlich nicht auf eine Behandlung mit einem CDK-Inhibitor anspricht.

**3.** Das Verfahren gemäß Anspruch 1, wobei der vorgegebene Referenzwert das MCL-1:BCL-xL-Genexpressionsverhältnis ist, das bei einer biologischen Probe erhalten wird, die Zellen von einem oder mehreren Patienten umfasst, bei dem/denen kein Krebs diagnostiziert wurde.

**4.** Das Verfahren gemäß Anspruch 1, wobei der vorgegebene Referenzwert das MCL-1:BCL-xL-Genexpressionsverhältnis ist, das bei einer biologischen Probe erhalten wird, die Zellen von einem oder mehreren Patienten umfasst, der/die krankheitsfrei ist/sind oder dessen/deren Zellen keine aberrante CDK-Signalisierung zeigt.

**5.** Das Verfahren gemäß Anspruch 1, wobei der Krebs ausgewählt ist aus der Gruppe bestehend aus akuter myeloischer Leukämie (AML), chronischer myeloischer Leukämie (CML), akuter lymphatischer Leukämie (ALL), chronischer lymphatischer Leukämie, Kaposi-Sarkom, Brustkrebs, Knochenkrebs, Gehirntumor, Kopf-Hals-Karzinom, Gallenblasen- und Gallengangkarzinomen, Netzhautkarzinomen, Speiseröhrenkarzinomen, Magenkrebs, multiplem Myelom, Ovarialkarzinom, Gebärmutterkarzinom, Schilddrüsenkrebs, Hodenkrebs, Endometriumkarzinom, Melanom, Kolorektalkarzinom, Blasenkrebs, Prostatakrebs, Lungenkrebs, Bauchspeicheldrüsenkrebs, Sarkome, Wilms-Tumor, Gebärmutterhalskrebs, Hautkrebs, Nasopharynxkarzinom, Liposarkom, epithelialem Karzinom, Nierenzellkarzinom, Gallenblasen-Adenokarzinom, Parotis-Adenokarzinom und Endometriumsarkom.

**6.** Ein CDK-Inhibitor zur Verwendung bei der Behandlung eines Krebspatienten, der durch das Verfahren gemäß einem der Ansprüche 1-5 als voraussichtlich ansprechend identifiziert wurde, wobei der CDK-Inhibitor ((S)-(-)--(-)2-(1-{3-Ethyl-7-[(1-oxypyridin-3-ylmethyl)]amino]pyrazolo[1,5-a]-pyrimidin-5-yl}piperidin-2-yl)ethanol)(Dinaciclib) oder ein pharmazeutisch annehmbares Salz davon ist.

## Revendications

**1.** Procédé d'identification d'un patient cancéreux susceptible de répondre à un traitement avec un inhibiteur de CDK, comprenant :

(a) mesurer la valeur d'un biomarqueur prédictif d'un inhibiteur de CDK dans un échantillon biologique obtenu d'un patient cancéreux ;
(b) comparer la valeur du biomarqueur à une valeur de référence prédéterminée pour déterminer si la valeur est au-dessus ou au-dessous de la valeur de référence prédéterminée ; et
(c) identifier un patient susceptible de répondre à un traitement avec un inhibiteur de CDK, où le biomarqueur prédictif est le rapport d'expression génique de MCL-1:BCL-xL et le patient répondeur a une valeur au-dessus de la valeur de

référence prédéterminée, où l'inhibiteur de CDK est du (S)-(-)--(-)2-(1-{3-éthyl-7-[(1-oxy-pyridin-3-ylméthyl)]amino]pyrazolo[1,5-a]pyrimidin-5-yl}pipéridin-2-yl)éthanol)(dinaciclib) ou un sel pharmaceutiquement acceptable de celui-ci, et le rapport d'expression génique de MCL-1:BCL-xL est le niveau d'ARNm.

**2.** Procédé selon la revendication 1, dans lequel un patient ayant un rapport d'expression génique de MCL-1:BCL-xL au-dessous de la valeur de référence est identifié comme un patient susceptible de ne pas répondre à un traitement avec un inhibiteur de CDK.

**3.** Procédé selon la revendication 1, dans lequel la valeur de référence prédéterminée est le rapport d'expression génique de MCL-1:BCL-xL obtenu d'un échantillon biologique comprenant des cellules provenant d'un ou de plusieurs patients qui n'ont pas été diagnostiqués avec un cancer.

**4.** Procédé selon la revendication 1, dans lequel la valeur de référence prédéterminée est le rapport d'expression génique de MCL-1:BCL-xL obtenu d'un échantillon biologique comprenant des cellules provenant d'un ou de plusieurs patients qui sont exempts de maladie ou dont les cellules ne présentent pas une signalisation aberrante de CDK.

**5.** Procédé selon la revendication 1, dans lequel le cancer est sélectionné parmi le groupe consistant en leucémie myéloïde aiguë (LMA), leucémie myéloïde chronique (LMC), leucémie aiguë lymphoblastique (LAL), leucémie chronique lymphoblastique, syndrome de Kaposi, cancer du sein, cancer des os, cancer du cerveau, cancer de la tête et du cou, cancers de la vésicule biliaire et du canal biliaire, cancers de la rétine, cancers de l'oesophage, cancer de l'estomac, myélome multiple, cancer de l'ovaire, cancer utérin, cancer de la thyroïde, cancer des testicules, cancer de l'endomètre, mélanome, cancer colorectal, cancer de la vessie, cancer de la prostate, cancer du poumon, cancer du pancréas, sarcomes, tumeur de Wilms, cancer du col de l'utérus, cancer de la peau, carcinome nasopharyngien, liposarcome, carcinome épithélial, carcinome des cellules rénales, adénocarcinome de la vésicule biliaire, adénocarcinome de la parotide et sarcome de l'endomètre.

**6.** Inhibiteur de CDK à utiliser dans le traitement d'un patient cancéreux identifié comme susceptible de répondre au procédé selon l'une quelconque des revendications 1-5, où l'inhibiteur de CDK est du ((S)-(-)--(-)2-(1-{3-éthyl-7-[(1-oxy-pyridin-3-ylméthyl)]amino]pyrazolo[1,5-a]pyrimidin-5-yl}pipéridin-2-yl)éthanol))(dinaciclib) ou un sel pharmaceutiquement acceptable de celui-ci.

Dinaciclib

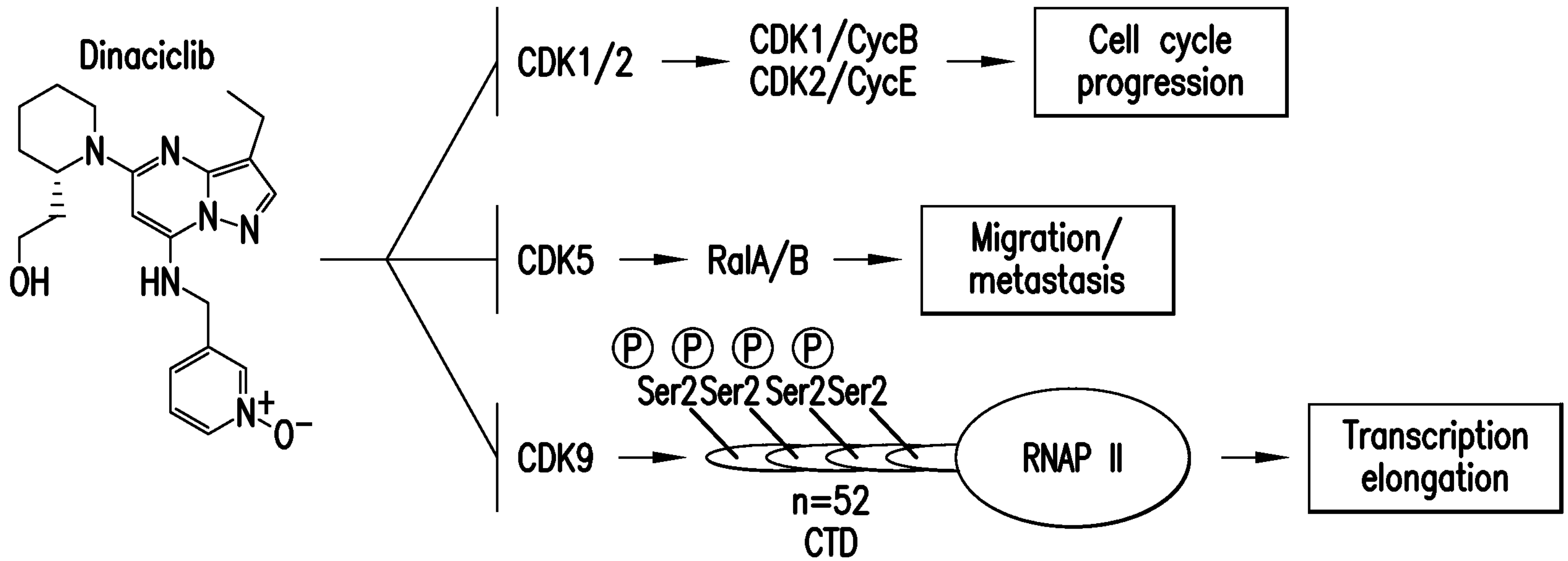


FIG.1

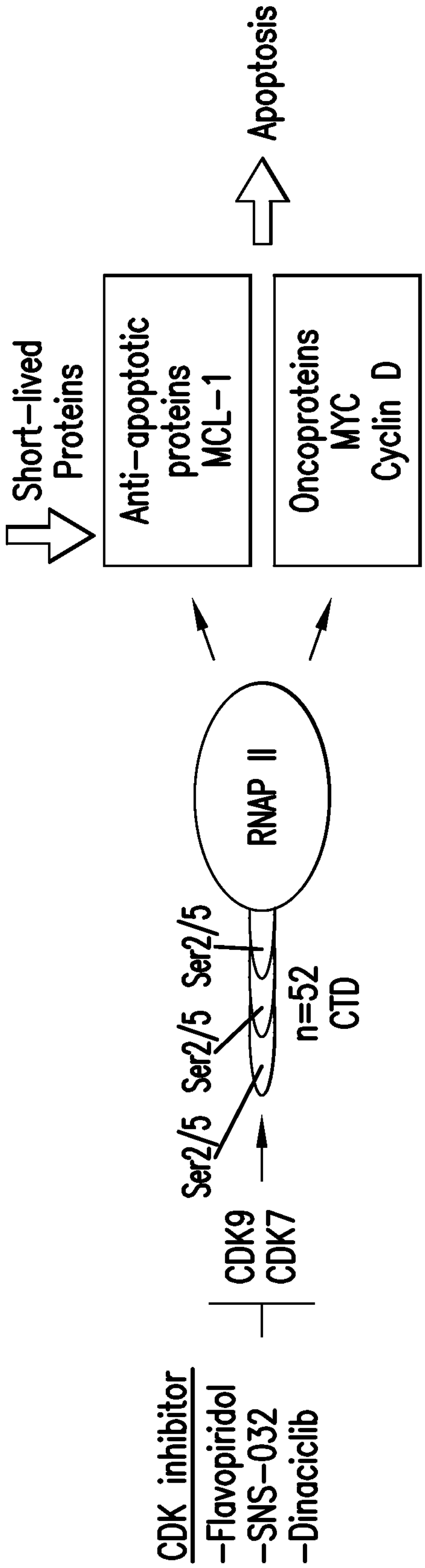
CDK inhibitor
-Flavopiridol
-SNS-032
-Dinaciclib
CDK9
CDK7
Ser2/5
Ser2/5
Ser2/5
n=52
CTD
RNAP II
Short-lived
Proteins
Anti-apoptotic
proteins
MCL-1
Oncoproteins
MYC
Cyclin D
Apoptosis

FIG. 2

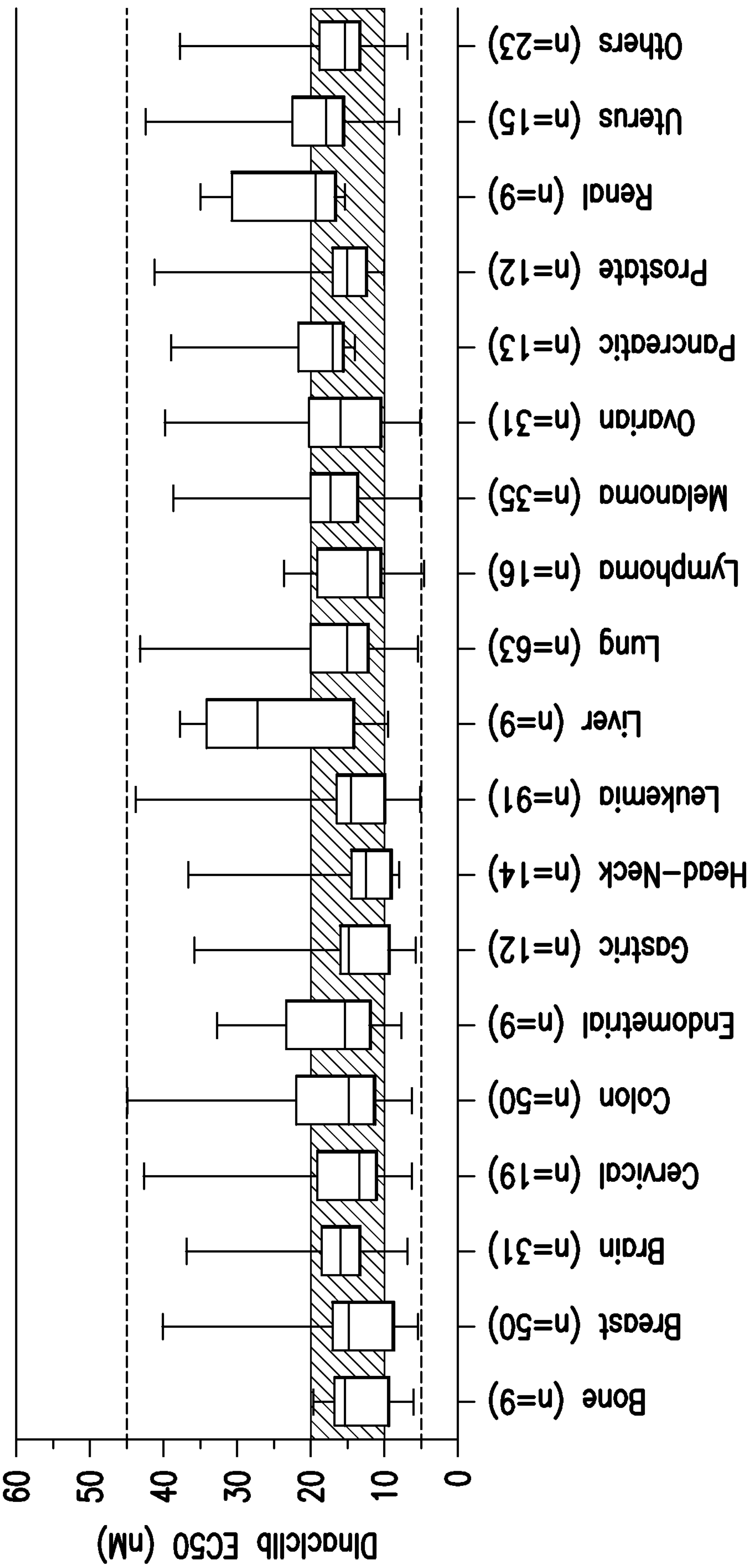
Bone (n=9)
Breast (n=50)
Brain (n=31)
Cervical (n=19)
Colon (n=50)
Endometrial (n=9)
Gastric (n=12)
Head–Neck (n=14)
Leukemia (n=91)
Liver (n=9)
Lung (n=63)
Lymphoma (n=16)
Melanoma (n=35)
Ovarian (n=31)
Pancreatic (n=13)
Prostate (n=12)
Renal (n=9)
Uterus (n=15)
Others (n=23)
Dinaciclib EC50 (nM)
0
10
20
30
40
50
60

FIG.3

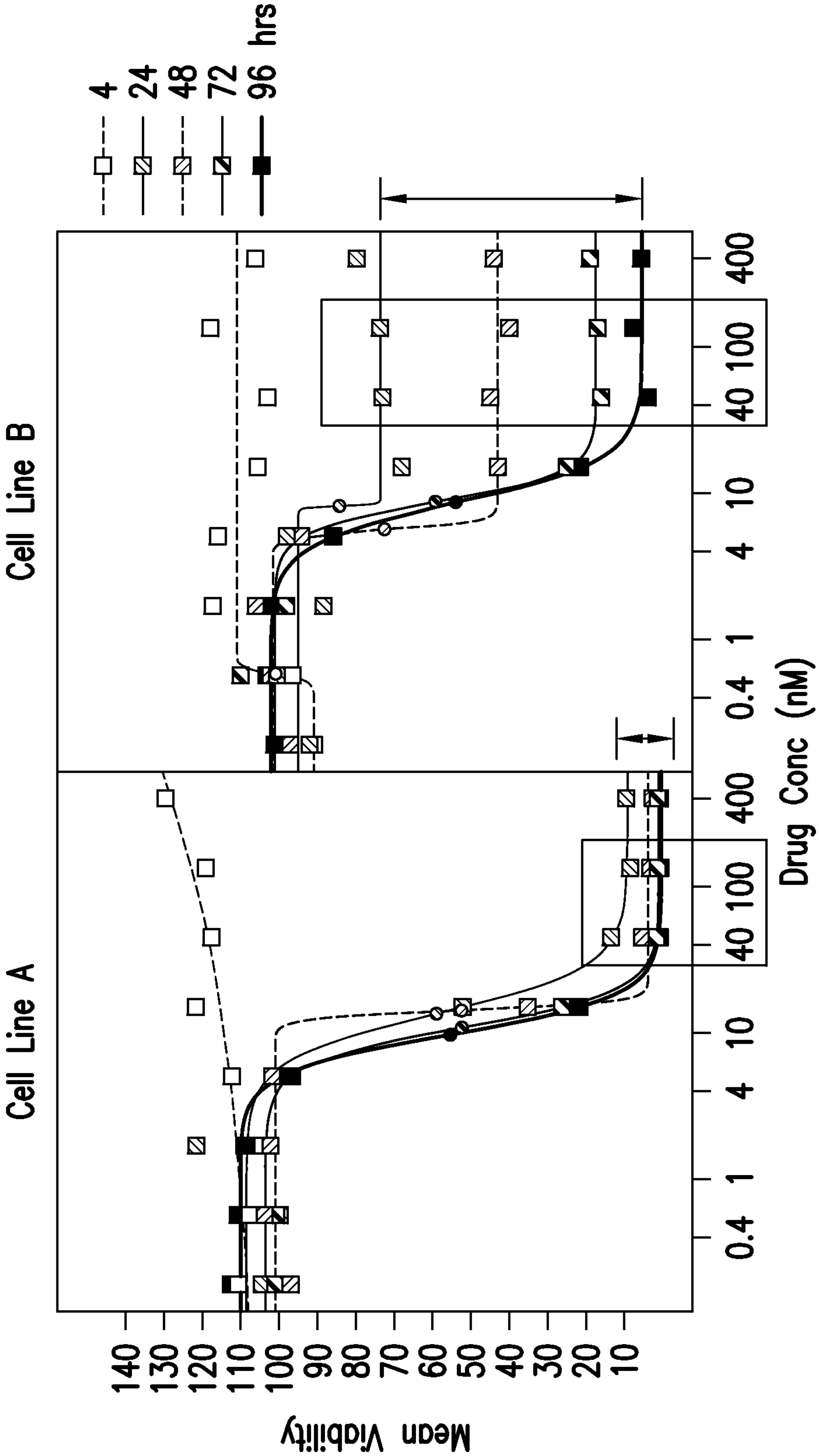
Cell Line A
Cell Line B
4
24
48
72
96 hrs
Mean Viability
Drug Conc (nM)

FIG.4

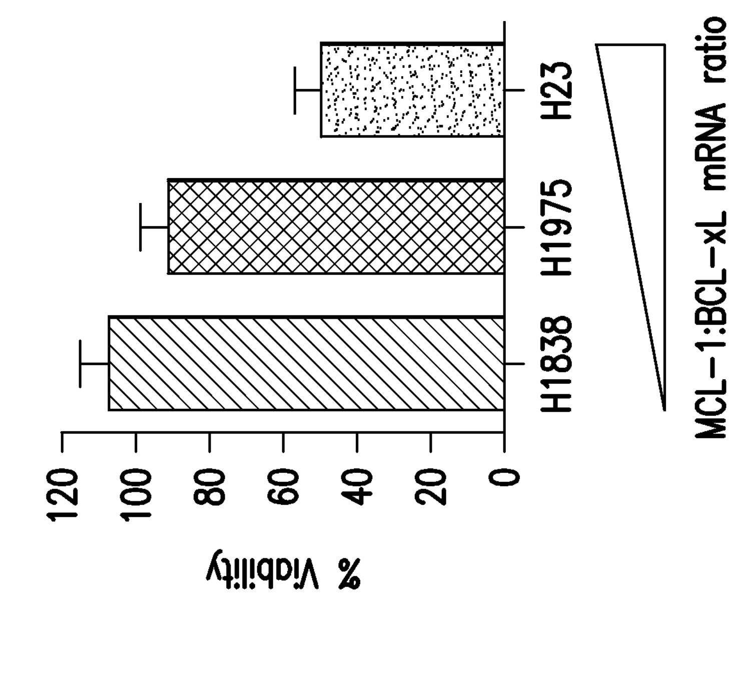
% Viability
120
100
80
60
40
20
0
H1838
H1975
H23
MCL-1:BCL-xL mRNA ratio

FIG.5A

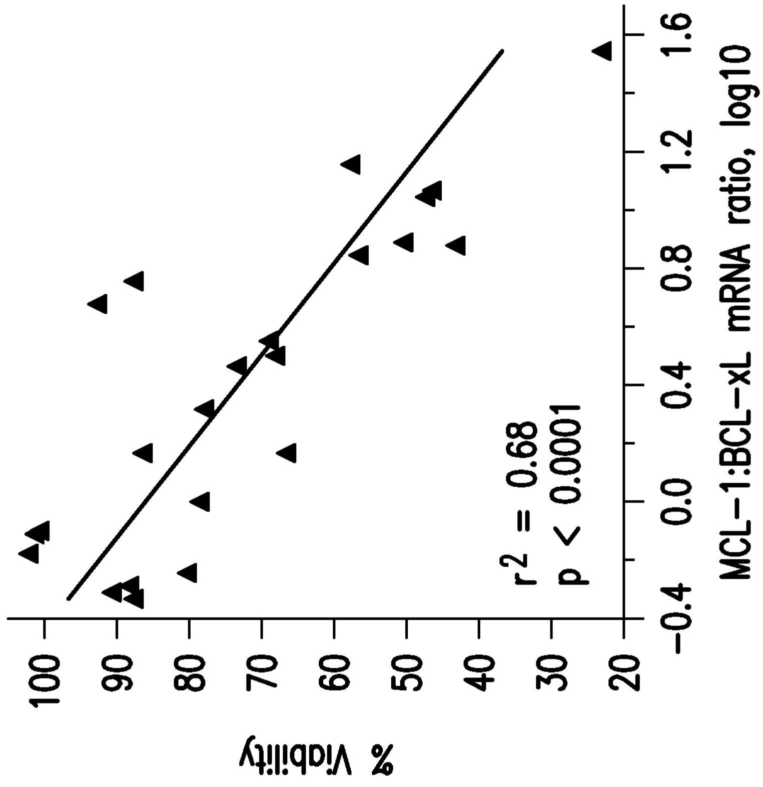
% Viability
100
90
80
70
60
50
40
30
20
r2 = 0.68
p < 0.0001
-0.4
0.0
0.4
0.8
1.2
1.6
MCL-1:BCL-xL mRNA ratio, log10

FIG.5B

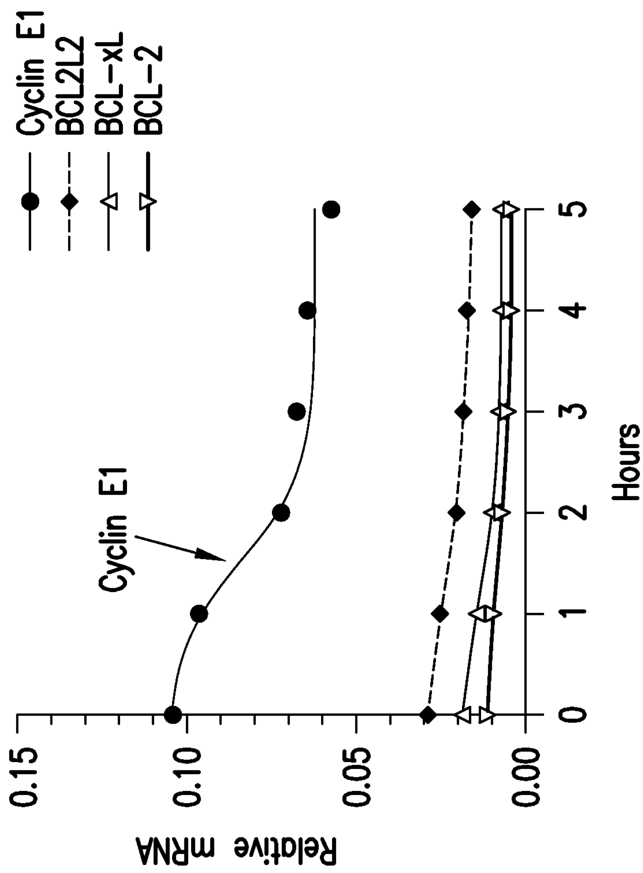
Cyclin E1
BCL2L2
BCL-xL
BCL-2
Cyclin E1
Relative mRNA
0.15
0.10
0.05
0.00
0
1
2
3
4
5
Hours

FIG.6B

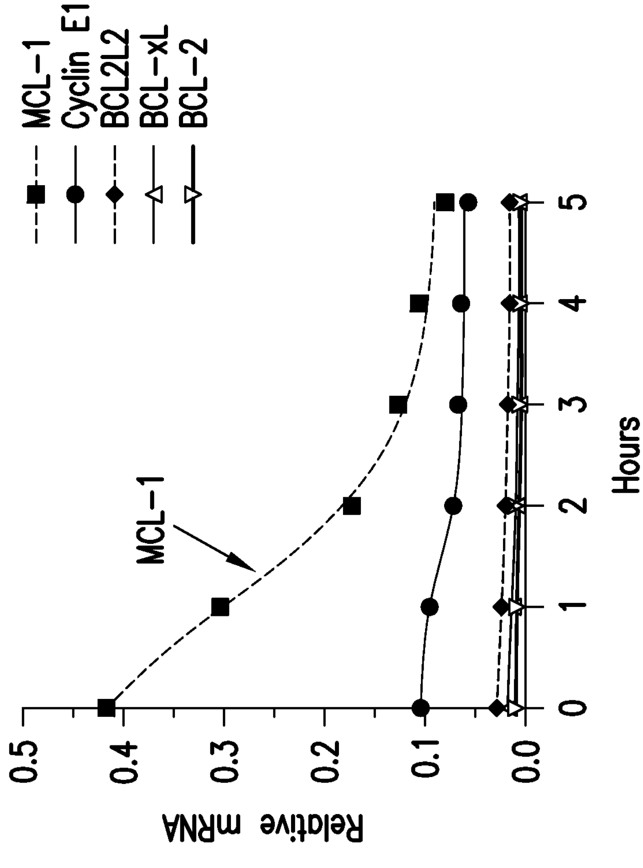
MCL-1
Cyclin E1
BCL2L2
BCL-xL
BCL-2
MCL-1
Relative mRNA
0.5
0.4
0.3
0.2
0.1
0.0
0
1
2
3
4
5
Hours

FIG.6A

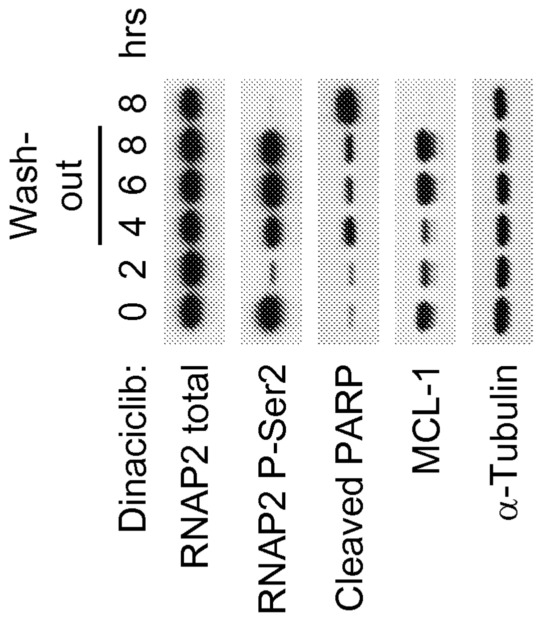
Dinaciclib: 0 2 4 6 8 8 hrs
Wash-out
RNAP2 total
RNAP2 P-Ser2
Cleaved PARP
MCL-1
α-Tubulin

FIG.7B

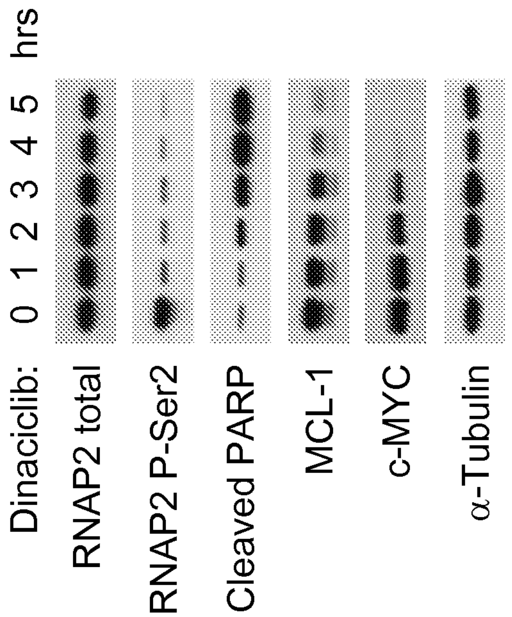
Dinaciclib: 0 1 2 3 4 5 hrs
RNAP2 total
RNAP2 P-Ser2
Cleaved PARP
MCL-1
c-MYC
α-Tubulin

FIG.7A

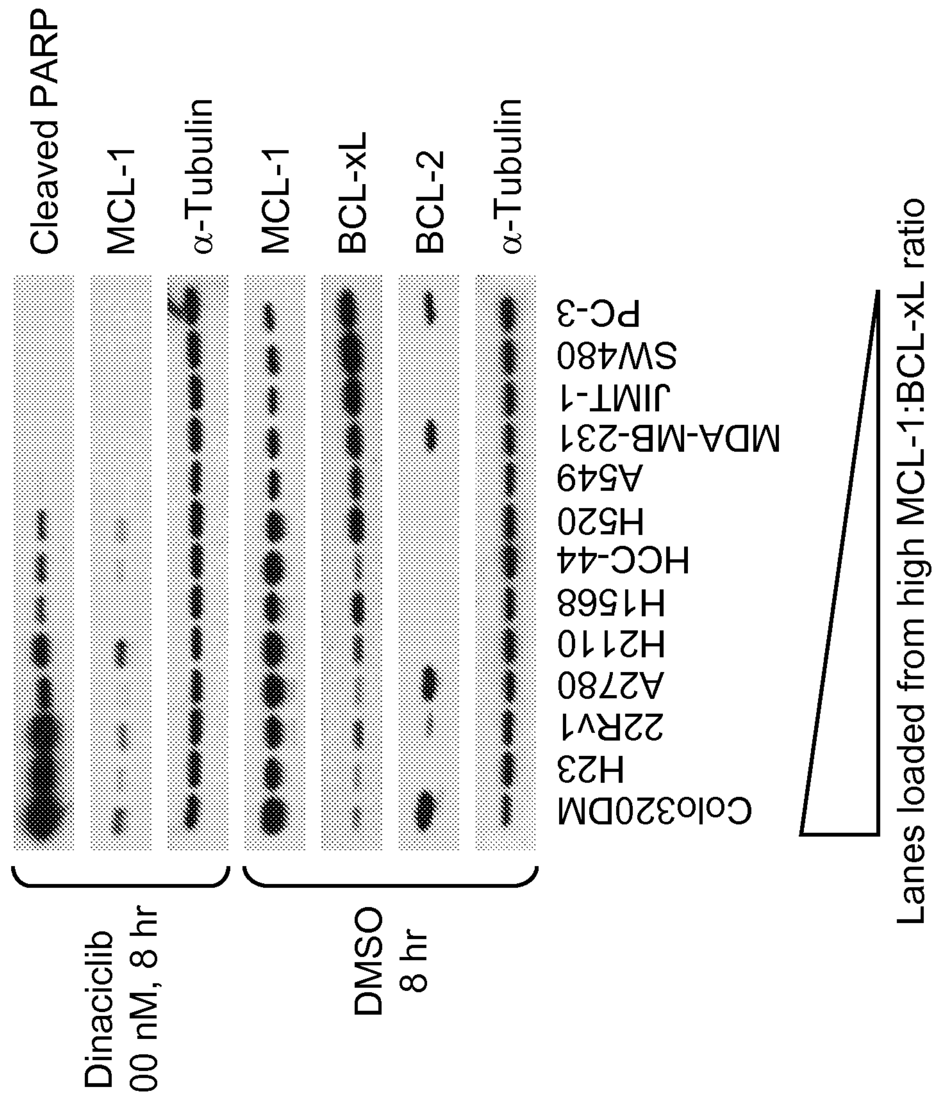
Cleaved PARP
MCL-1
α-Tubulin
MCL-1
BCL-xL
BCL-2
α-Tubulin
Dinaciclib
100 nM, 8 hr
DMSO
8 hr
PC-3
SW480
JIMT-1
MDA-MB-231
A549
H520
HCC-44
H1568
H2110
A2780
22Rv1
H23
Colo320DM
Lanes loaded from high MCL-1:BCL-xL ratio

FIG.8

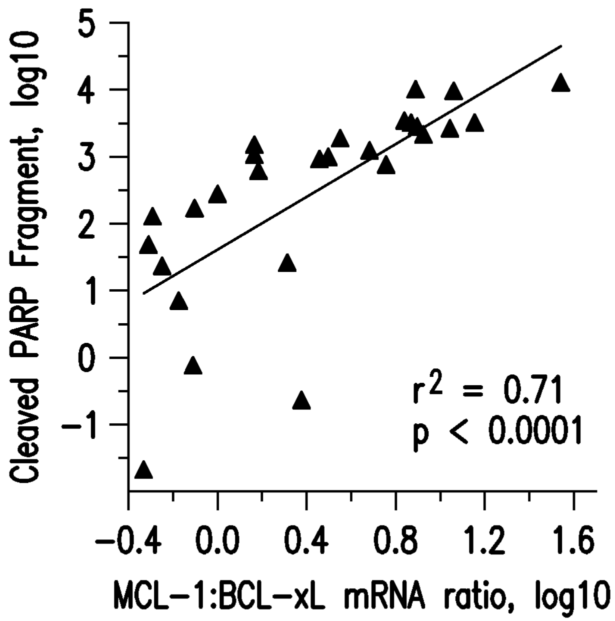
Cleaved PARP Fragment, log10
MCL-1:BCL-xL mRNA ratio, log10
$r^2$ = 0.71
p < 0.0001
5
4
3
2
1
0
−1
−0.4
0.0
0.4
0.8
1.2
1.6

FIG.9A

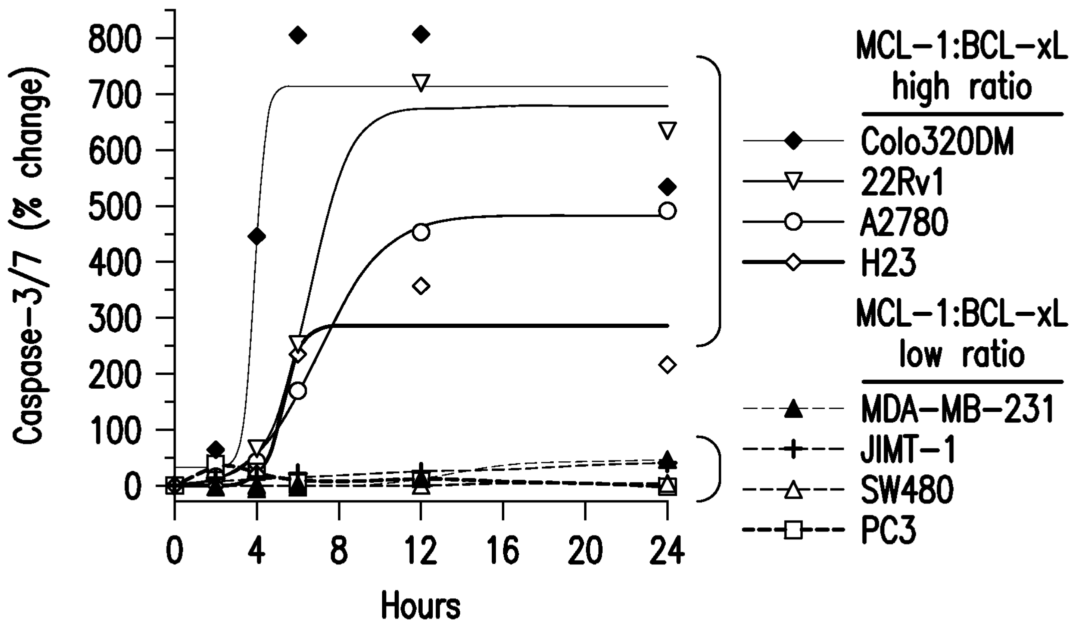
Caspase-3/7 (% change)
Hours
800
700
600
500
400
300
200
100
0
0
4
8
12
16
20
24
MCL-1:BCL-xL high ratio
Colo320DM
22Rv1
A2780
H23
MCL-1:BCL-xL low ratio
MDA-MB-231
JIMT-1
SW480
PC3

FIG.9B

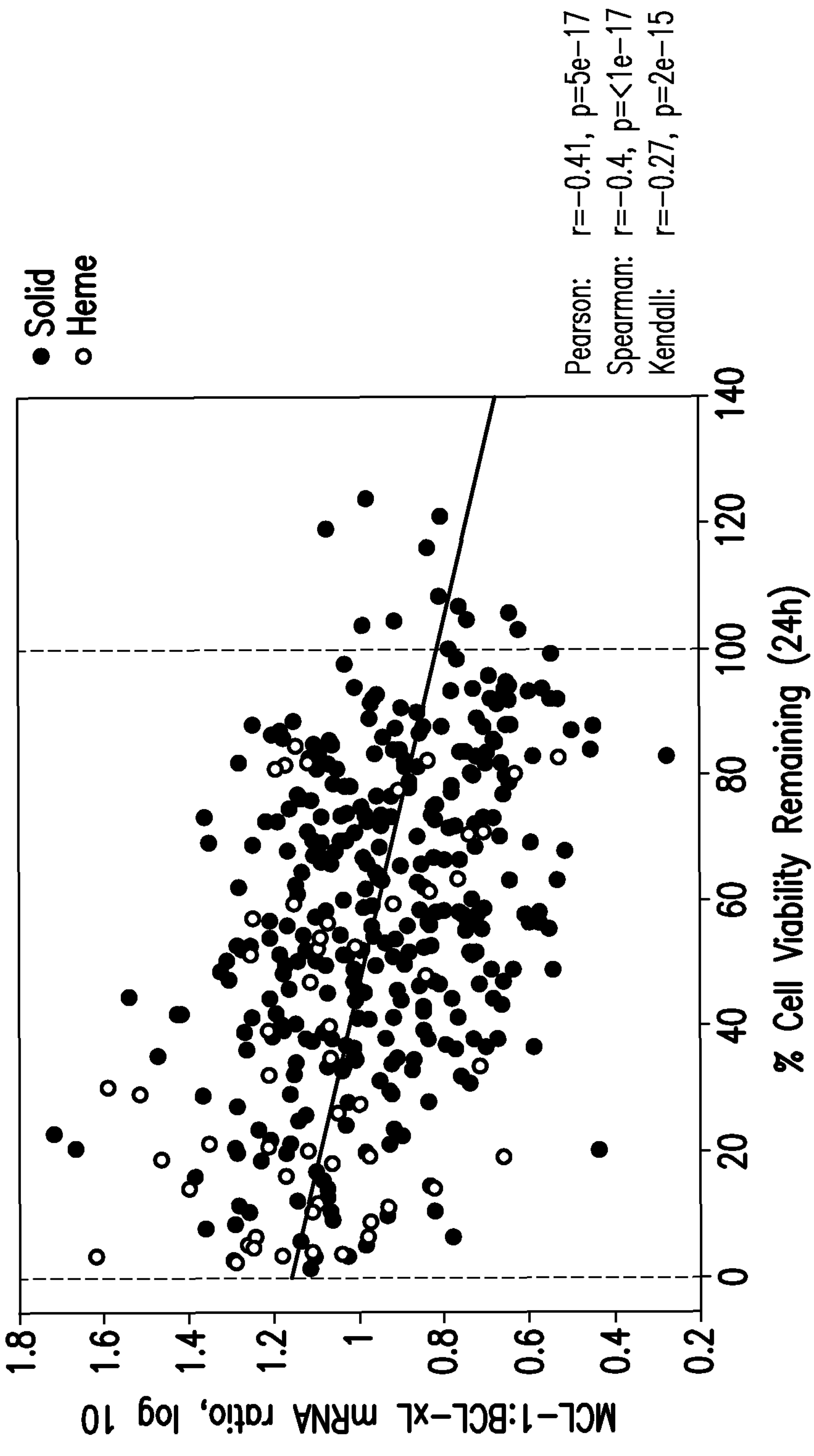

● Solid
o Heme
Pearson: r=−0.41, p=5e−17
Spearman: r=−0.4, p=<1e−17
Kendall: r=−0.27, p=2e−15
MCL−1:BCL−xL mRNA ratio, log 10
1.8
1.6
1.4
1.2
1
0.8
0.6
0.4
0.2
% Cell Viability Remaining (24h)
0
20
40
60
80
100
120
140


FIG.10

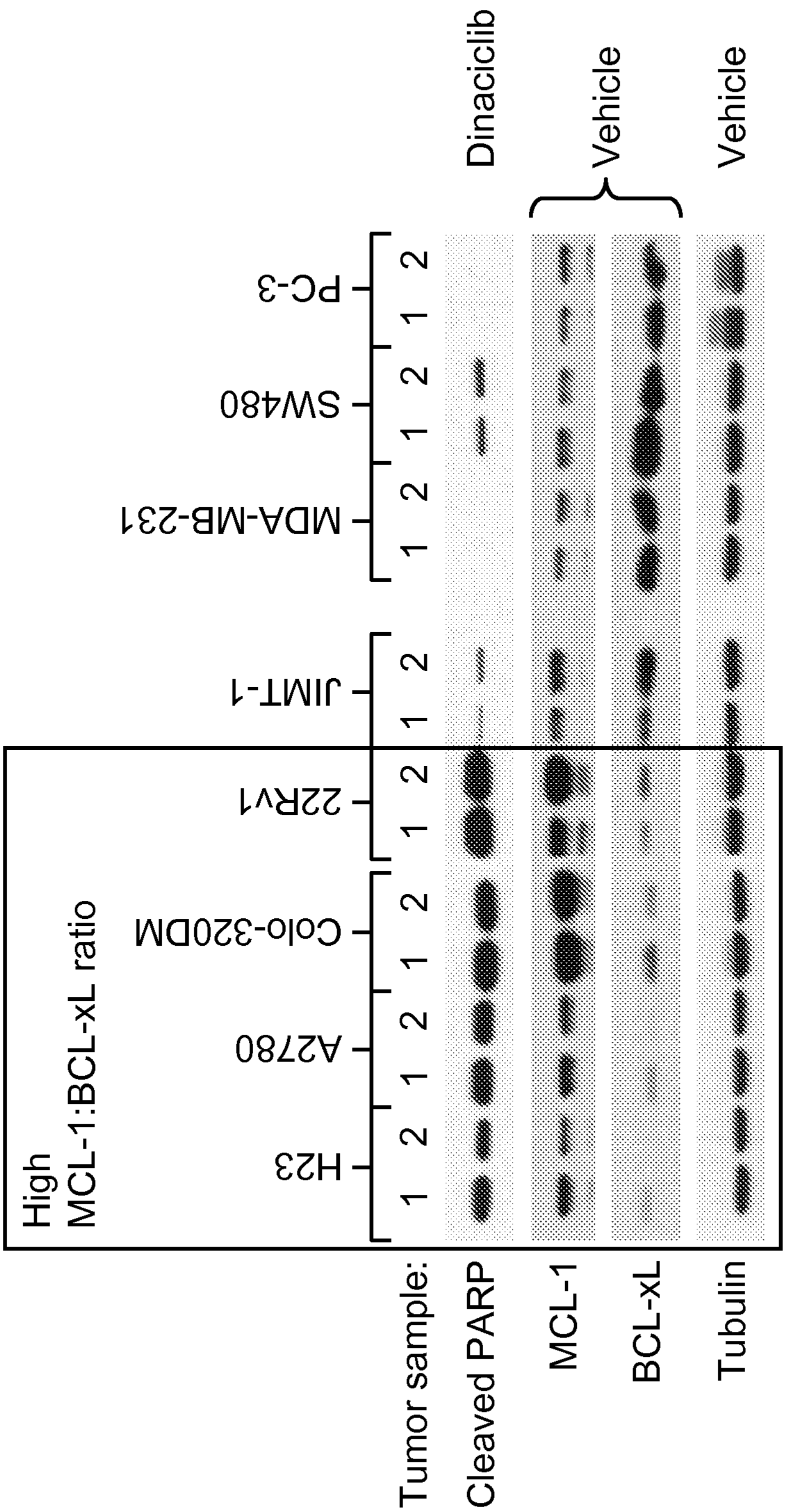
Tumor sample:
Cleaved PARP
MCL-1
BCL-xL
Tubulin
High
MCL-1:BCL-xL ratio
H23
A2780
Colo-320DM
22Rv1
JIMT-1
MDA-MB-231
SW480
PC-3
1
2
Dinaciclib
Vehicle
Vehicle

FIG.11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0222610 A1, M. Hale
- US 6413974 B
- US 6107305 A
- WO 0210162 A
- US 7119200 B
- US 5569588 A
- WO 2007076128 A
- WO 2007076129 A
- US 7171311 B
- WO 2002103320 A
- WO 200508689L A
- WO 20060L5312 A
- WO 2006084272 A
- US 20030104426 A, Similarly, Linsley
- US 20070154931 A, Radish
- US 6551784 B
- US 5807522 A
- US 4683202 A
- US 5242974 A
- US 5384261 A
- US 5405783 A
- US 5412087 A
- US 5424186 A
- US 5429807 A
- US 5436327 A
- US 5445934 A
- US 5556752 A
- US 5472672 A
- US 5527681 A
- US 5529756 A
- US 5545531 A
- US 5554501 A
- US 5561071 A
- US 5571639 A
- US 5593839 A
- US 5624711 A
- US 5700637 A
- US 5744305 A
- US 5770456 A
- US 5770722 A
- US 5837832 A
- US 5856101 A
- US 5874219 A
- US 5885837 A
- US 5919523 A
- US 6022963 A
- US 6077674 A
- US 6156501 A
- US 5143854 A
- US 5288644 A
- US 5324633 A
- US 5432049 A
- US 5470710 A
- US 5492806 A
- US 5503980 A
- US 5510270 A
- US 5525464 A
- US 5547839 A
- US 5580732 A
- US 5661028 A
- US 5848659 A
- US 5631734 A
- WO 2007126122 A
- WO 2007126128 A
- WO 2008133866 A


### Non-patent literature cited in the description


- **Y. METTEY et al.** *J. Med. Chem.,* 2003, vol. 46, 222-236
- **A. M. SANDEROWICZ et al.** *J. Clin. Oncol.,* 1998, vol. 16, 2986-2999
- **J. VESELY et al.** *Eur. J. Biochem.,* 1994, vol. 224, 771-786
- **L. MEIJER et al.** *Eur. J. Biochem.,* 1997, vol. 243, 527-536
- **K. S. KIM et al.** *J. Med. Chem.,* 2002, vol. 45, 3905-3927
- **A. MANDELIN ; R. POPE.** *Expert Opin. Ther. Targets,* 2007, vol. 11 (3), 363-373
- *Mandelin and Pope,* 2007, 367-368
- **F.T. AWAN et al.** *Blood,* 2009, vol. 113 (3), 535-537
- **GEORG FELDMANN et al.** Cyclin-dependent kinase inhibitor Dinaciclib (SCH727965) inhibits pancreatic cancer growth and progression in murine xenograft models. *CANCER BIOLOGY & THERAPY,* 01 October 2011, vol. 12 (7), 598-609
- **R. CHEN et al.** *Blood,* 2005, vol. 106 (7), 2513-2519
- **R. CHEN et al.** *Blood,* 2009, vol. 113 (19), 4637-4645

- **D. PARRY et al.** *Mol. Cancer Ther.,* 2010, vol. 9 (8), 2344-235
- **W. FU et al.** *Mol. Cancer Ther.,* 2011, vol. 10 (6), 1018-1027
- **VELCULESCU et al.** *Science,* 1995, vol. 270, 484-87
- **TIAN et al.** *Nucleic Acids Res.,* 2004, vol. 32, e126
- **KIM et al.** *Science,* 2007, vol. 316, 1481-84
- **DASL ; BIBIKOVA et al.** *AJP,* 2004, vol. 165, 1799-807
- **FLAGELLA et al.** *Anal. Biochem.,* 2006, vol. 352, 50-60
- **CHEN et al.** *Blood,* 2005, vol. 106, 2513-2519
- **CHEN et al.** *Blood,* 2009, vol. 113, 4637-4645
- **BEROUKHIM et al.** *Nature,* 2010, vol. 463, 899-905
- **ZHANG et al.** *Oncogene,* 2011, vol. 30, 1963-1968
- **D. PARRY et al.** *Mol. Cancer Ther.,* 2010, vol. 9 (8), 2344-2353
- **R. CHEN et al.** *Blood,* 2005, vol. 106 (7), 2513-2516
- **SAMBROOK et al.** MOLECULAR CLONING--A LABORATORY MANUAL. Cold Spring Harbor Laboratory, 1989, vol. 1 3
- **AUSUBEL et al.** CURRENT PROTOCOLS IN MOLECULAR BIOLOGY. Current Protocols Publishing, 1994, vol. 2
- **CHIRGWIN et al.** *Biochemistry,* 1979, vol. 18, 5294-5299
- Life Testing. S-PLUS 2000 GUIDE TO STATISTICS. 2000, vol. 2, 368
- **KONONEN et al.** *Nat. Med,* 1998, vol. 4 (7), 844-847
- **AUSUBEL et al.** Current Protocols of Molecular Biology. John Wiley and Sons, 1997
- PCR Strategies. 1995
- **C. R. NEWTON ; A. GRAHAM.** *PCR: Introduction to Biotechniques Series,* 1997
- **KIM et al.** *J. Virol.,* 1992, vol. 66, 3879-3882
- **BISWAS et al.** *Ann. NY Acad. Sci.,* 1990, vol. 590, 582-583
- **BISWAS et al.** *J. Clin. Microbiol.,* 1991, vol. 29, 2228-2233
- **BONNER et al.** *Science,* 1997, vol. 278, 1481
- **EMMERT-BUCK et al.** *Science,* 1996, vol. 274, 998
- **FEND et al.** *Am. J. Path.,* 1999, vol. 154, 61
- **MURAKAMI et al.** *Kidney Hit.,* 2000, vol. 58, 1346
- **DULAC.** *Curr. Top. Dev. Biol.,* 1998, vol. 36, 245
- **JENA et al.** *J. Immunol. Methods,* 1996, vol. 190, 199
- **WANG et al.** *Proc. Natl. Acad. Sci. USA,* 1989, vol. 86, 9717
- **WU ; WALLACE.** *Genomics,* 1989, vol. 4, 560
- **LANDEGREN et al.** *Science,* 1988, vol. 241, 1077
- **GUATELLI et al.** *Proc. Natl. Acad. Sci. USA,* 1990, vol. 87, 1874
- **KWOH et al.** *Proc. Natl. Acad. Sci. USA,* 1989, vol. 86, 1173
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 4967
- **ECKERT et al.** *PCR Methods and Applications,* 1991, vol. 1, 17
- PCR. IRL Press
- **OHYAMA et al.** *BioTechniques,* 2000, vol. 29, 530
- **LUO et al.** *Nat. Med.,* 1999, vol. 5, 117
- **HEGDE et al.** *BioTechniques,* 2000, vol. 29, 548
- **KACHARMINA et al.** *Meth. Enzymol.,* 1999, vol. 303, 3
- **LIVESEY et al.** *Curr. Biol.,* 2000, vol. 10, 301
- **SPIRIN et al.** *Invest. Ophtalmol. Vis. Sci.,* 1999, vol. 40, 3108
- **SAKAI et al.** *Anal. Biochem.,* 2000, vol. 287, 32
- **EBERWINE et al.** *Proc. Natl. Acad. Sci. USA,* 1992, vol. 89, 3010
- **KRICKA.** Nonisotopic DNA Probe Techniques. Academic Press, 1992
- **TYAGI ; KRAMER.** *Nature Biotech.,* 1996, vol. 14, 303
- **THIEL et al.** *Anal. Chem.,* 1997, vol. 69, 4948
- **SHENA et al.** *Science,* 1995, vol. 270, 467-470
- **SHENA et al.** *Tibtech,* 1998, vol. 16, 301
- **DUGGAN et al.** *Nat.Genet.,* 1999, vol. 21, 10
- **BOWTELL et al.** *Nat. Genet.,* 1999, vol. 21, 25
- **LIPSHUTZ et al.** *Nature Genet.,* 1999, vol. 21, 20-24
- **BLANCHARD et al.** *Biosensors and Bioelectronics,* 1996, vol. 11, 687-690
- **MASKOS et al.** *Nucleic Acids Res.,* 1993, vol. 21, 4663-4669
- **HUGHES et al.** *Nat. Biotechol.,* 2001, vol. 19, 342
- Hybridization With Nucleic Acid Probes. **TIJSSEN et al.** Laboratory Techniques in Biochemistry and Molecular Biology. Elsevier, 1993, vol. 24